# EUROPEAN PATENT APPLICATION

(11) **EP 1 371 661 A1**
(43) Date of publication of application: **17.12.2003**
(21) Application number: 02013098.5
(22) Date of filing: 13.06.2002
(51) Int. Cl.: C07K 14/705, C12N 15/12, C12N 15/62, C12N 15/10, C12Q 1/68, G01N 33/68, G01N 33/50

(54) **Variants of the Constitutive Activated of Retinoid acid response receptor (CAR) in its ligand binding domain (LBD)**

(71) Applicant: LION bioscience AG, 69123 Heidelberg (DE)
(72) Inventor: Jackson, David, 69120 Heidelberg (DE); Kranz, Harald, 69181 Leimen (DE); Kögl, Manfred, 69214 Eppelheim (DE); Picker, Alexander, 69126 Heidelberg (DE)
(74) Representative: Goddar, Heinz J., Dr.

(57) **Abstract**

The present invention provides, *inter alia*, novel nuclear receptor proteins. In a preferred embodiment of the invention novel CAR homologues are provided. Also provided are the nucleic acid sequences encoding these novel nuclear receptor proteins. The CAR homologues are useful for developing and identifying compounds for the treatment of such diseases and disorders as metabolic disorders, immunological indications, hormonal dysfunctions, neuro-systemic diseases and in preferred embodiments, high cholesterol and artheriosclerosis as well as other metabolic disorders.

SEQUENCE LISTING

<110>  LION bioscience AG
<120>  Variants in the ligand binding domain of the nuclear receptor CAR
<130>  FB11149
<160>  20
<170>  PatentIn version 3.1

<210>  1
<211>  1113
<212>  DNA
<213>  Homo sapiens

<400>  1
atggccagta gggaagatga gctgaggaac tgtgtggtat gtggggacca agccacaggc      60

taccacttta atgcgctgac ttgtgagggc tgcaagggtt tcttcaggag aacagtcagc     120

aaaagcattg gtcccacctg cccctttgct ggaagctgtg aagtcagcaa gactcagagg     180

cgccactgcc cagcctgcag gttgcagaag tgcttagatg ctggcatgag gaaagacatg     240

atactgtcgg cagaagccct ggcattgcgg cgagcaaagc aggcccagcg gcgggcacag     300

caaacacctg tgcaactgag taaggagcaa gaagagctga tccggacact cctgggggcc     360

cacacccgcc acatgggcac catgtttgaa cagtttgtgc agtttaggcc tccagctcat     420

ctgttcatcc atcaccaccc cttgcccacc ctggcccctg tgctgcctct ggtcacacac     480

ttcgcagaca tcaacacttt catggtactg caagtcatca agtttactaa ggacctgcct     540

gtcttccgtt ccctgcccat tgaagaccag atctcccttc tcaagggagc agctgtggaa     600

atctgtcaca tcgtactcaa taccactttc tgtctccaaa cacaaaactt cctctgcggg     660

cctcttcgct acacaattga agatggagcc cgtgtatctc ccacagtggg gttccaggta     720

gagtttttgg agttgctctt tcacttccat ggaacactac gaaaactgca gctccaagag     780

cctgagtatg tgctcttggc tgccatggcc ctcttctctc ctgaccgacc tggagttacc     840

cagagagatg agattgatca gctgcaagag gagatggcac tgactctgca aagctacatc     900

aagggccagc agcgaaggcc ccgggatcgg tttctgtatg cgaagttgct aggcctgctg     960

gctgagctcc ggagcattaa tgaggcctac gggtaccaaa tccagcacat ccagggcctg    1020

tctgccatga tgccgctgct ccaggagatc tgcagctgag gccatgctca cttccttccc    1080

cagctcacct ggaacaccct ggatacactg gag                                 1113


<210>  2
<211>  1113
<212>  DNA
<213>  Homo sapiens
<400>  2
atggccagta gggaagatga gctgaggaac tgtgtggtat gtggggacca agccacaggc      60

taccacttta atgcgctgac ttgtgagggc tgcaagggtt tcttcaggag aacagtcagc     120

aaaagcattg gtcccacctg cccctttgct ggaagctgtg aagtcagcaa gactcagagg     180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccagcc cttgcccacc ctggcccctg tgctgcctct ggtcacacac 480

ttcgcagaca tcaacacttt catggtactg caagtcatca agtttactaa ggacctgccc 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc ctggggttcc aggtagagtt tttggagttg 720

ctctttcact tccatggaac actacgaaaa ctgcagctcc aagagcctga gtatgtgctc 780

ttggctgcca tggccctctt ctctcctgct ccctatctta cagaccgacc tggagttacc 840

cagagagatg agattgatca gctgcaagag gagatggcac tgactctgca aagctacatc 900

aagggccagc agcgaaggcc ccgggatcgg tttctgtatg cgaagttgct aggcctgctg 960

gctgagctcc ggagcattaa tgaggcctac gggtaccaaa tccagcacat ccagggcctg 1020

tctgccatga tgccgctgct ccaggagatc tgcagctgag gccatgctca cttccttccc 1080

cagctcacct ggaacaccct ggatacactg gag 1113

<210> 3
<211> 1128
<212> DNA
<213> Homo sapiens
<400> 3
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccagcc cttgcccacc ctggcccctg tgctgcctct ggtcacacac 480

ttcgcagaca tcaacacttt catggtactg caagtcatca agtttactaa ggacctgcct 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtatctc ccacagtggg gttccaggta 720

gagtttttgg agttgctctt tcacttccat ggaacactac gaaaactgca gctccaagag 780

cctgagtatg tgctcttggc tgccatggcc ctcttctctc ctgctcccta tcttacagac 840

cgacctggag ttacccagag agatgagatt gatcagctgc aagaggagat ggcactgact 900

ctgcaaagct acatcaaggg ccagcagcga aggccccggg atcggtttct gtatgcgaag 960

ttgctaggcc tgctggctga gctccggagc attaatgagg cctacgggta ccaaatccag 1020

cacatccagg gcctgtctgc catgatgccg ctgctccagg agatctgcag ctgaggccat 1080

gctcacttcc ttccccagct cacctggaac accctggata cactggag 1128

<210> 4
<211> 1116
<212> DNA
<213> Homo sapiens
<400> 4
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccagcc cttgcccacc ctggcccctg tgctgcctct ggtcacacac 480

ttcgcagaca tcaacacttt catggtactg caagtcatca agtttactaa ggacctgcct 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtggggt tccaggtaga gtttttggag 720

ttgctctttc acttccatgg aacactacga aaactgcagc tccaagagcc tgagtatgtg 780

ctcttggctg ccatggccct cttctctcct gctccctatc ttacagaccg acctggagtt 840

acccagagag atgagattga tcagctgcaa gaggagatgg cactgactct gcaaagctac 900

atcaagggcc agcagcgaag gccccgggat cggtttctgt atgcgaagtt gctaggcctg 960

ctggctgagc tccggagcat taatgaggcc tacgggtacc aaatccagca catccagggc 1020

ctgtctgcca tgatgccgct gctccaggag atctgcagct gaggccatgc tcacttcctt 1080

ccccagctca cctggaacac cctggataca ctggag 1116

<210> 5
<211> 947
<212> DNA
<213> Homo sapiens

<400> 5
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccaccc cttgcccacc ctggcccctg tgctgcctct ggtcacacac 480

ttcgcagaca tcaacacttt catggtactg caagtcatca agtttactaa ggacctgccc 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtggggt tccaggtaga gtttttggag 720

ttgctctttc acttccatgg aacactacga aaactgcagc tccaagagcc tgagtatgtg 780

ctcttggctg ccatggccct cttctctcct gaccgacctg gagttaccca gagagatgag 840

attgatcagc tgcaagagga gatggcactg actctgcaaa gctacatcaa gggccagcag 900

cgaaggcccc gggatcgctc acctggaaca ccctggatac actggag 947

<210> 6
<211> 1101
<212> DNA
<213> Homo sapiens
<400> 6
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccaccc cttgcccacc ctggcccctg tgctgcctct ggtcacacac 480

ttcgcagaca tcaacacttt catggtactg caagtcatca agtttactaa ggacctgccc 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtggggt tccaggtaga gtttttggag 720

ttgctctttc acttccatgg aacactacga aaactgcagc tccaagagcc tgagtatgtg 780

ctcttggctg ccatggccct cttctctcct gaccgacctg gagttaccca gagagatgag 840

attgatcagc tgcaagagga gatggcactg actctgcaaa gctacatcaa gggccagcag 900

cgaaggcccc gggatcggtt tctgtatgcg aagttgctag gcctgctggc tgagctccgg 960

agcattaatg aggcctacgg gtaccaaatc cagcacatcc agggcctgtc tgccatgatg 1020

ccgctgctcc aggagatctg cagctgaggc catgctcact tccttcccca gctcacctgg 1080

aacaccctgg atacactgga g 1101

<210> 7
<211> 999
<212> DNA
<213> Homo sapiens
<400> 7
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccagcc cttgcccacc ctggcccctg tgctgcctct ggtcacacac 480

ttcgcagaca tcaacacttt catggtactg caagtcatca agtttactaa ggacctgccc 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgctccct atcttacaga ccgacctgga 720

gttacccaga gagatgagat tgatcagctg caagaggaga tggcactgac tctgcaaagc 780

tacatcaagg gccagcagcg aaggccccgg gatcggtttc tgtatgcgaa gttgctaggc 840

ctgctggctg agctccggag cattaatgag gcctacgggt accaaatcca gcacatccag 900

ggcctgtctg ccatgatgcc gctgctccag gagatctgca gctgaggcca tgctcacttc 960

cttccccagc tcacctggaa caccctggat acactggag 999

<210> 8
<211> 1040
<212> DNA
<213> Homo sapiens
<400> 8
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccaccc cttgcccacc ctggcccctg tgctgcctct ggtcacacac 480

ttcgcagaca tcaacacttt catggtactg caagtcatca agtttactaa ggacctgcct 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtggggt tccaggtaga gtttttggag 720

ttgctctttc acttccatgg aacactacga aaactgcagc tccaagagcc tgagtatgtg 780

ctcttggctg ccatggccct cttctctcct gctccctatc ttacagaccg acctggagtt 840

acccagagag atgagattga tcagctgcaa gaggagatgg cactgactct gcaaagctac 900

atcaagggcc agcagcgaag gccccgggat cgcacatcca gggcctgtct gccatgatgc 960

cgctgctcca ggagatctgc agctgaggcc atgctcactt ccttccccag ctcacctgga 1020

acaccctgga tacactggag 1040

<210> 9
<211> 845
<212> DNA
<213> Homo sapiens
<400> 9
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccaccc cttgcccacc ctggcccctg tgctgcctct ggtcacacac 480

ttcgcagaca tcaacacttt catggtactg caagtcatca agtttactaa ggacctgccc 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgctccct atcttacaga ccgacctgga 720

gttacccaga gagatgagat tgatcagctg caagaggaga tggcactgac tctgcaaagc 780

tacatcaagg gccagcagcg aaggccccgg gatcgctcac ctggaacacc ctggatacac 840

tggag 845

<210> 10
<211> 947
<212> DNA
<213> Homo sapiens
<400> 10
atggccagta gggaagatga gctgaggaac tgtgtggtat gtggggacca agccacaggc 60

taccacttta atgcgctgac ttgtgagggc tgcaagggtt tcttcaggag aacagtcagc 120

aaaagcattg gtcccacctg cccctttgct ggaagctgtg aagtcagcaa gactcagagg 180

cgccactgcc cagcctgcag gttgcagaag tgcttagatg ctggcatgag gaaagacatg 240

atactgtcgg cagaagccct ggcattgcgg cgagcaaagc aggcccagcg gcgggcacag 300

caaacacctg tgcaactgag taaggagcaa gaagagctga tccggacact cctgggggcc 360

cacacccgcc acatgggcac catgtttgaa cagtttgtgc agtttaggcc tccagctcat 420

ctgttcatcc atcaccagcc cttgcccacc ctggcccctg tgctgcctct ggtcacacac 480

ttcgcagaca tcaacacttt catggtactg caagtcatca agtttactaa ggacctgcct 540

gtcttccgtt ccctgcccat tgaagaccag atctcccttc tcaagggagc agctgtggaa 600

atctgtcaca tcgtactcaa taccactttc tgtctccaaa cacaaaactt cctctgcggg 660

cctcttcgct acacaattga agatggagcc cgtgtggggt tccaggtaga gtttttggag 720

ttgctctttc acttccatgg aacactacga aaactgcagc tccaagagcc tgagtatgtg 780

ctcttggctg ccatggccct cttctctcct gaccgacctg gagttaccca gagagatgag 840

attgatcagc tgcaagagga gatggcactg actctgcaaa gctacatcaa gggccagcag 900

cgaaggcccc gggatcgctc acctggaaca ccctggatac actggag 947

<210> 11
<211> 352
<212> PRT
<213> Homo sapiens
<400> 11

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His His Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Ser Pro Thr Val Gly Phe Gln Val
225                 230                 235                 240

Glu Phe Leu Glu Leu Leu Phe His Phe His Gly Thr Leu Arg Lys Leu
                245                 250                 255

Gln Leu Gln Glu Pro Glu Tyr Val Leu Leu Ala Ala Met Ala Leu Phe
            260                 265                 270

Ser Pro Asp Arg Pro Gly Val Thr Gln Arg Asp Glu Ile Asp Gln Leu
        275                 280                 285

Gln Glu Glu Met Ala Leu Thr Leu Gln Ser Tyr Ile Lys Gly Gln Gln
    290                 295                 300

Arg Arg Pro Arg Asp Arg Phe Leu Tyr Ala Lys Leu Leu Gly Leu Leu
305                 310                 315                 320

Ala Glu Leu Arg Ser Ile Asn Glu Ala Tyr Gly Tyr Gln Ile Gln His
                325                 330                 335

Ile Gln Gly Leu Ser Ala Met Met Pro Leu Leu Gln Glu Ile Cys Ser
            340                 345                 350

<210>  12
<211>  352
<212>  PRT
<213>  Homo sapiens

<400>  12

Met Ala Ser Arg Glu Asp Glu Leu Arg Asn Cys Val Val Cys Gly Asp

1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His Gln Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Leu Gly Phe Gln Val Glu Phe Leu Glu Leu
225                 230                 235                 240

Leu Phe His Phe His Gly Thr Leu Arg Lys Leu Gln Leu Gln Glu Pro
                245                 250                 255

Glu Tyr Val Leu Leu Ala Ala Met Ala Leu Phe Ser Pro Ala Pro Tyr
            260                 265                 270

Leu Thr Asp Arg Pro Gly Val Thr Gln Arg Asp Glu Ile Asp Gln Leu
        275                 280                 285

Gln Glu Glu Met Ala Leu Thr Leu Gln Ser Tyr Ile Lys Gly Gln Gln
    290                 295                 300

Arg Arg Pro Arg Asp Arg Phe Leu Tyr Ala Lys Leu Leu Gly Leu Leu
305                 310                 315                 320

Ala Glu Leu Arg Ser Ile Asn Glu Ala Tyr Gly Tyr Gln Ile Gln His
                325                 330                 335

Ile Gln Gly Leu Ser Ala Met Met Pro Leu Leu Gln Glu Ile Cys Ser

340                 345                 350

<210>  13
<211>  357
<212>  PRT
<213>  Homo sapiens

<400>  13

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His Gln Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Ser Pro Thr Val Gly Phe Gln Val
225                 230                 235                 240

Glu Phe Leu Glu Leu Leu Phe His Phe His Gly Thr Leu Arg Lys Leu
                245                 250                 255

Gln Leu Gln Glu Pro Glu Tyr Val Leu Leu Ala Ala Met Ala Leu Phe
            260                 265                 270

Ser Pro Ala Pro Tyr Leu Thr Asp Arg Pro Gly Val Thr Gln Arg Asp
        275                 280                 285

Glu Ile Asp Gln Leu Gln Glu Glu Met Ala Leu Thr Leu Gln Ser Tyr
    290                 295                 300

Ile Lys Gly Gln Gln Arg Arg Pro Arg Asp Arg Phe Leu Tyr Ala Lys
305                 310                 315                 320

Leu Leu Gly Leu Leu Ala Glu Leu Arg Ser Ile Asn Glu Ala Tyr Gly
                325                 330                 335

Tyr Gln Ile Gln His Ile Gln Gly Leu Ser Ala Met Met Pro Leu Leu
            340                 345                 350

Gln Glu Ile Cys Ser
        355

<210>  14
<211>  353
<212>  PRT
<213>  Homo sapiens

<400>  14

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His Gln Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Gly Phe Gln Val Glu Phe Leu Glu
225                 230                 235                 240

Leu Leu Phe His Phe His Gly Thr Leu Arg Lys Leu Gln Leu Gln Glu
                245                 250                 255

Pro Glu Tyr Val Leu Leu Ala Ala Met Ala Leu Phe Ser Pro Ala Pro
            260                 265                 270

Tyr Leu Thr Asp Arg Pro Gly Val Thr Gln Arg Asp Glu Ile Asp Gln
        275                 280                 285

Leu Gln Glu Glu Met Ala Leu Thr Leu Gln Ser Tyr Ile Lys Gly Gln
    290                 295                 300

Gln Arg Arg Pro Arg Asp Arg Phe Leu Tyr Ala Lys Leu Leu Gly Leu
305                 310                 315                 320

Leu Ala Glu Leu Arg Ser Ile Asn Glu Ala Tyr Gly Tyr Gln Ile Gln
                325                 330                 335

His Ile Gln Gly Leu Ser Ala Met Met Pro Leu Leu Gln Glu Ile Cys
            340                 345                 350

Ser

<210>  15
<211>  315
<212>  PRT
<213>  Homo sapiens

<400>  15

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His

130                 135                 140

His His Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Gly Phe Gln Val Glu Phe Leu Glu
225                 230                 235                 240

Leu Leu Phe His Phe His Gly Thr Leu Arg Lys Leu Gln Leu Gln Glu
                245                 250                 255

Pro Glu Tyr Val Leu Leu Ala Ala Met Ala Leu Phe Ser Pro Asp Arg
            260                 265                 270

Pro Gly Val Thr Gln Arg Asp Glu Ile Asp Gln Leu Gln Glu Glu Met
        275                 280                 285

Ala Leu Thr Leu Gln Ser Tyr Ile Lys Gly Gln Gln Arg Arg Pro Arg
    290                 295                 300

Asp Arg Ser Pro Gly Thr Pro Trp Ile His Trp
305                 310                 315

<210>  16
<211>  348
<212>  PRT
<213>  Homo sapiens

<400>  16

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His His Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Gly Phe Gln Val Glu Phe Leu Glu
225                 230                 235                 240

Leu Leu Phe His Phe His Gly Thr Leu Arg Lys Leu Gln Leu Gln Glu
                245                 250                 255

Pro Glu Tyr Val Leu Leu Ala Ala Met Ala Leu Phe Ser Pro Asp Arg
            260                 265                 270

Pro Gly Val Thr Gln Arg Asp Glu Ile Asp Gln Leu Gln Glu Glu Met
        275                 280                 285

Ala Leu Thr Leu Gln Ser Tyr Ile Lys Gly Gln Gln Arg Arg Pro Arg
    290                 295                 300

Asp Arg Phe Leu Tyr Ala Lys Leu Leu Gly Leu Leu Ala Glu Leu Arg
305                 310                 315                 320

Ser Ile Asn Glu Ala Tyr Gly Tyr Gln Ile Gln His Ile Gln Gly Leu
                325                 330                 335

Ser Ala Met Met Pro Leu Leu Gln Glu Ile Cys Ser
            340                 345


<210>  17
<211>  314
<212>  PRT
<213>  Homo sapiens

<400>  17

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His Gln Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Ala Pro Tyr Leu Thr Asp Arg Pro Gly
225                 230                 235                 240

Val Thr Gln Arg Asp Glu Ile Asp Gln Leu Gln Glu Glu Met Ala Leu
                245                 250                 255

Thr Leu Gln Ser Tyr Ile Lys Gly Gln Gln Arg Arg Pro Arg Asp Arg
            260                 265                 270

Phe Leu Tyr Ala Lys Leu Leu Gly Leu Leu Ala Glu Leu Arg Ser Ile
        275                 280                 285

Asn Glu Ala Tyr Gly Tyr Gln Ile Gln His Ile Gln Gly Leu Ser Ala
    290                 295                 300

Met Met Pro Leu Leu Gln Glu Ile Cys Ser
305                 310


<210>  18
<211>  318
<212>  PRT
<213>  Homo sapiens

<400>  18

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys

            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His His Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Gly Phe Gln Val Glu Phe Leu Glu
225                 230                 235                 240

Leu Leu Phe His Phe His Gly Thr Leu Arg Lys Leu Gln Leu Gln Glu
                245                 250                 255

Pro Glu Tyr Val Leu Leu Ala Ala Met Ala Leu Phe Ser Pro Ala Pro
            260                 265                 270

Tyr Leu Thr Asp Arg Pro Gly Val Thr Gln Arg Asp Glu Ile Asp Gln
        275                 280                 285

Leu Gln Glu Glu Met Ala Leu Thr Leu Gln Ser Tyr Ile Lys Gly Gln
    290                 295                 300

Gln Arg Arg Pro Arg Asp Arg Thr Ser Arg Ala Cys Leu Pro
305                 310                 315

<210> 19
<211> 281
<212> PRT
<213> Homo sapiens

<400> 19

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His His Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Ala Pro Tyr Leu Thr Asp Arg Pro Gly
225                 230                 235                 240

Val Thr Gln Arg Asp Glu Ile Asp Gln Leu Gln Glu Glu Met Ala Leu
                245                 250                 255

Thr Leu Gln Ser Tyr Ile Lys Gly Gln Gln Arg Arg Pro Arg Asp Arg
            260                 265                 270

Ser Pro Gly Thr Pro Trp Ile His Trp
        275                 280

<210>  20
<211>  315
<212>  PRT
<213>  Homo sapiens

<400>  20

Met Ala Ser Arg Glu Asp Glu Leu Arg Asn Cys Val Val Cys Gly Asp
1               5                   10                  15

Gln Ala Thr Gly Tyr His Phe Asn Ala Leu Thr Cys Glu Gly Cys Lys
            20                  25                  30

Gly Phe Phe Arg Arg Thr Val Ser Lys Ser Ile Gly Pro Thr Cys Pro
        35                  40                  45

Phe Ala Gly Ser Cys Glu Val Ser Lys Thr Gln Arg Arg His Cys Pro
    50                  55                  60

Ala Cys Arg Leu Gln Lys Cys Leu Asp Ala Gly Met Arg Lys Asp Met
65                  70                  75                  80

Ile Leu Ser Ala Glu Ala Leu Ala Leu Arg Arg Ala Lys Gln Ala Gln
                85                  90                  95

Arg Arg Ala Gln Gln Thr Pro Val Gln Leu Ser Lys Glu Gln Glu Glu
            100                 105                 110

Leu Ile Arg Thr Leu Leu Gly Ala His Thr Arg His Met Gly Thr Met
        115                 120                 125

Phe Glu Gln Phe Val Gln Phe Arg Pro Pro Ala His Leu Phe Ile His
    130                 135                 140

His Gln Pro Leu Pro Thr Leu Ala Pro Val Leu Pro Leu Val Thr His
145                 150                 155                 160

Phe Ala Asp Ile Asn Thr Phe Met Val Leu Gln Val Ile Lys Phe Thr
                165                 170                 175

Lys Asp Leu Pro Val Phe Arg Ser Leu Pro Ile Glu Asp Gln Ile Ser
            180                 185                 190

Leu Leu Lys Gly Ala Ala Val Glu Ile Cys His Ile Val Leu Asn Thr
        195                 200                 205

Thr Phe Cys Leu Gln Thr Gln Asn Phe Leu Cys Gly Pro Leu Arg Tyr
    210                 215                 220

Thr Ile Glu Asp Gly Ala Arg Val Gly Phe Gln Val Glu Phe Leu Glu
225                 230                 235                 240

Leu Leu Phe His Phe His Gly Thr Leu Arg Lys Leu Gln Leu Gln Glu
                245                 250                 255

Pro Glu Tyr Val Leu Leu Ala Ala Met Ala Leu Phe Ser Pro Asp Arg
            260                 265                 270

Pro Gly Val Thr Gln Arg Asp Glu Ile Asp Gln Leu Gln Glu Glu Met
        275                 280                 285

Ala Leu Thr Leu Gln Ser Tyr Ile Lys Gly Gln Gln Arg Arg Pro Arg
    290                 295                 300

Asp Arg Ser Pro Gly Thr Pro Trp Ile His Trp
305                 310                 315

## Description

### BACKGROUND OF THE INVENTION

For the development of a new pharmaceutical active substance, it is important that the interactions of the new substance with other medicaments are as low as possible. Interactions, amongst others, are elicited by the increased expression of gene products that are involved in the metabolism and excretion of xenobiotic substances. Enzymes like P450 cytochrome, glutathion-S-transferase or glucuronolyltransferases as well as transporters like MDR1, MDR2 and Oatps belong to this family of de-toxification enzymes. The determination of the induction of these de-toxification enzymes in cells of the liver is an important method for the prediction of interactions with medicaments.

Many of these signals produce their effects by ultimately changing the transcription of specific genes. One well-studied group of proteins that mediate a cell's response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known (Escriva, H. et al., Ligand binding was acquired during evolution of nuclear receptors, PNAS, 94, 6803 - 6808, 1997). Orphan receptors may be indicative of unknown signaling pathways in the cell or may be nuclear receptors that function without ligand activation. The activation of transcription by some of these orphan receptors may occur in the absence of an exogenous ligand and/or through signal transduction pathways originating from the cell surface (Mangelsdorf, D. J. et al., The nuclear receptor superfamily: the second decade, Cell 83, 835-839, 1995).

In general, three functional domains have been defined in NRs. An amino terminal domain is believed to have some regulatory function. A DNA-binding domain hereinafter referred to as "DBD" usually comprises two zinc finger elements and recognizes a specific Hormone Responsive Element hereinafter referred to as "HRE" within the promoters of responsive genes.

Specific amino acid residues in the "DBD" have been shown to confer DNA sequence binding specificity (Schena, M. & Yamamoto, K.R., Mammalian Glucocorticoid Receptor Derivatives Enhance Transcription in Yeast, Science, 241:965-967, 1988). A Ligand-binding-domain hereinafter referred to as "LBD" is at the carboxy-terminal region of known NRs. In the absence of hormone, the LBD appears to interfere with the interaction of the DBD with its HRE. Hormone binding seems to result in a conformational change in the NR and thus opens this interference (Brzozowski et al., Molecular basis of agonism and antagonism in the oestogen receptor, Nature, 389, 753 - 758, 1997; Wagner et al., A structural role for hormone in the thyroid hormone receptor, Nature, 378, 690 - 697. 1995). A NR without the hormone binding domain (herein referred to as "HBD") constitutively activates transcription, but at a low level.

Both the amino-terminal domain and the LBD appear to have transcription activation functions hereinafter referred to as "TAF". Acidic residues in the amino-terminal domains of some nuclear receptors may be important for these transcription factors to interact with RNA polymerase. TAF activity may be dependent on interactions with other protein factors or nuclear components (Diamond et al., Transcription Factor Interactions: Selectors of Positive or Negative Regulation from a Single DNA Element, Science, 249:1266-1272 , 1990). Certain onco-proteins (e.g., c-Jun and c-Fos) can show synergistic or antagonistic activity with glucocorticoid receptors (GR) in transfected cells. Furthermore, the receptors for estrogen and vitamins A and D, and fatty acids have been shown to interact, either physically or functionally, with the Jun and Fos components of AP-1 in the transactivation of steroid- or AP-1 regulated genes.

Coactivators or transcriptional activators are proposed to bridge between sequence specific transcription factors, the basal transcription machinery and in addition to influence the chromatin structure of a target cell. Several proteins like SRC-1, ACTR, and Grip1 interact with NRs in a ligand enhanced manner (Heery et al., A signature motif in transcriptional coactivators mediates binding to nuclear receptors, Nature, 387, 733 - 736; Heinzel et al., A complex containing N-CoR, mSin3 and histone deacetylase mediates transcriptional repression, Nature 387, 43 - 47, 1997). Furthermore, the physical interaction with negative receptor-interacting proteins or corepressors has been demonstrated (Xu et al., Coactivator and Corepressor complexes in nuclear receptor function, Curr Opin Genet Dev, 9 (2), 140 - 147, 1999).

Nuclear receptor modulators like steroid hormones affect the growth and function of specific cells by binding to intracellular receptors and forming nuclear receptor-ligand complexes. Nuclear receptor-hormone complexes then interact with a hormone response element (HRE) in the control region of specific genes and alter specific gene expression.

The regulation of the detox-enzymes is largely controlled by two nuclear receptors: PXR and CAR. These two regulators bind to a variety of different substances, are thereby activated and turn on the expression of the detox-enzymes (see, for example, Kast H. R. et al. Regulation of multidrug resistance-associated protein 2 (ABCC2) by the nuclear receptors pregnane X receptor, famesoid X-activated receptor, and constitutive androstane receptor. J Biol Chem. 2002; 277(4):2908-15. and Toell, A. et al. Orphan nuclear receptor bionding site in the human inducible nitric oxide synthase promoter mediates responsiveness to steroid and xenobiotic ligands; J. Cell. Biochem. 2002; 85(1): 72-82). Therefore, in a general method, the effect of substances on PXR and CAR can be determined in order to predict potential interactions. The latter method uses the known gene products of PXR and CAR in assays for the activity determination.

Through its regulatory function in detox mechanisms, an additional CAR homologue could serve as a target for cholesterol lowering drugs and exert beneficial effects in diseases like artheriosclerosis and other metabolic disorders, inflammation and apoptosis. Furthermore, since not all variants of CAR are known yet, it was impossible to elucidate the effects of CAR modulating substances on the individual isoforms of the receptor, thus making a reliable prediction impossible. The disadvantage is that there can be alternative products of the CAR and PXR-genes which exhibit a different spectrum of binding due to alternative splicing in the LBD. Compounds tested against only one variant of the receptor could nevertheless induce another variant of the same receptor.

It was thus an object of the present invention to provide for novel CAR nuclear receptor variants, which exhibit a different binding behaviour of substances. In a preferred embodiment of the invention it was an object to provide for homologues of the LBD of CAR. It was an object of the present invention to provide for means of producing these receptors as well as means of screening for agonists and antagonists to the receptors. Further objects of the invention are outlined below.

### SUMMARY OF THE INVENTION

The present invention provides, *inter alia,* novel nuclear receptor proteins. In a preferred embodiment of the invention novel CAR homologues are provided. Also provided are the nucleic acid sequences encoding these novel nuclear receptor proteins, as well as compounds and methods for using these protein and their nucleic acid sequence.

The present invention provides a novel proteins, nucleic acids, and methods useful for developing and identifying compounds for the treatment of such diseases and disorders as metabolic disorders, immunological indications, hormonal dysfunctions, neurosystemic diseases and in preferred embodiments, high cholesterol and artheriosclerosis as well as other metabolic disorders.

Identified and disclosed herein is the protein sequence for novel nuclear receptor variants and the nucleic acid sequences encoding these nuclear receptors, which are designated herein as "CAR-spl" or simply "CAR variant", or "CARs".

The importance of this discovery is manifested in the effects of CARs to modulate genes involved in cellular functions, like the detoxification.

Thus, this CARs proteins are useful for screening for CARs agonists and antagonist activity for controlling these conditions. The present receptors will behave surprisingly different in terms of pairing, interaction with other proteins and interacting compounds (ligands), and therefore their biological functions. Thus, these receptors will enable a whole new area of applications with respect to the development and identification of compounds for the production of medicaments.

In one aspect of the present invention, we provide isolated nucleic acid sequences for novel receptors, the CARs. In particular, we provide the cDNA sequences, protein sequences as well as the genomic sequences encoding the human CARs.

These nucleic acid sequences have a variety of uses. For example, they are useful for making vectors and for transforming cells, both of which are ultimately useful for production of the CARs.

They are also useful as scientific research tools for developing nucleic acid probes for determining CARs expression levels, e.g., to identify diseased or otherwise abnormal states. They are useful for developing analytical tools such as anti sense oligonucleotides for selectively inhibiting expression of the CARs genes to determine physiological responses.

In another aspect of the present invention, we provide a homogenous composition comprising the CARs protein. The protein is useful for screening drugs for agonist and antagonist activity, and, therefore, for screening for drugs useful in regulating physiological responses associated with CARs. Specifically, antagonists to the CARs receptor could be used to treat diseases and disorders as metabolic disorders, disorders in the detoxification, whereas agonists could be used for the treatment of these conditions. The proteins are also useful for developing antibodies for detection of the protein.

Flowing from the foregoing are a number of other aspects of the invention, including (a) vectors, such as plasmids, comprising the CARs nuclear receptor nucleic acid sequence that may further comprise additional regulatory elements, *e.g.*, promoters, (b) transformed cells that express the CARs, (c) nucleic acid probes, (d) antisense oligonucleotides, (e) agonists, (f) antagonists, and (g) transgenic mammals. Further aspects of the invention comprise methods for making and using the foregoing compounds and compositions.

The foregoing merely summarizes certain aspects of the present invention and is not intended, nor should it be construed, to limit the invention in any manner. All patents and other publications recited herein are hereby incorporated by reference in their entirety.

### DETAILED DESCRIPTION OF THE PREFERRED EMBODIMENTS

### THE CARS PROTEIN AND NUCLEIC ACID:

The present invention comprises novel members of the CAR nuclear receptor superfamily which the inventors herein refer to as CARs. Particularly preferred embodiments of the CARs are those having an amino acid sequence substantially the same as SEQ ID NOs. 11 to 20. The analysis of the amino acid sequence confirms that the present protein is indeed a member of the nuclear receptor family which is closely related to CAR.

In view of the above, a major aspect of the present invention is the provision of a polypeptide of CARs receptor, comprising the amino acid sequence according to SEQ ID NOs. 11 to 20, or having an amino acid sequence substantially the same or similar as SEQ ID NOs. 11 to 20.

As used herein, a protein "having an amino acid sequence substantially the same or similar as SEQ ID NO x" (where "x" is the number of one of the protein sequences recited in the Sequence Listing) means a protein whose amino acid sequence is the same as SEQ ID NO x or differs only in a way such that at least 50% of the residues compared in a sequence alignment with SEQ ID NO. x are identical, preferably 75% of the residues are identical, even more preferably 95% of the residues are identical and most preferably at least 98% of the residues are identical.

Those skilled in the art will appreciate that conservative substitutions of amino acids can be made without significantly diminishing the protein's affinity for interacting proteins, DNA binding sites, CARs receptor modulators, e.g. small molecular hydrophobic compounds, or RNA.

Other substitutions may be made that increase the protein's affinity for these compounds. Making and identifying such proteins is a routine matter given the teachings herein, and can be accomplished, for example, by altering the nucleic acid sequence encoding the protein (as disclosed herein), inserting it into a vector, transforming a cell, expressing the nucleic acid sequence, and measuring the binding affinity of the resulting protein, all as taught herein.

Another aspect of the present invention is directed to a fusion protein comprising a polypeptide according to the present invention. Such fusion proteins are described in more detail below. In addition, the polypeptide according to the present invention can be a synthetically produced polypeptide. Again, the way of producing such polypeptides is further described below.

As used herein, if reference to CAR-spl, the CARs receptor, the nuclear receptor CARs or the CARs nuclear receptor is made it is meant as a reference to any protein having an amino acid sequence substantially the same as SEQ ID NOs. 11 to 20.

The CAR receptor proteins provided by the present invention are of mammalian, more preferably human origin.

The nucleic acids claimed according to the present invention may be present in various forms, i.e. as an RNA, DNA, cDNA or as genomic DNA. In particular, the present invention is related to a nucleic acid encoding for a polypeptide according to the invention, wherein said nucleic acid is genomic DNA, cDNA, immature mRNA or mature mRNA, in particular the nucleic acid sequence according to SEQ ID NOs. 1 to 10, and optionally comprises at least one suitable label. Furthermore, the sequence of the inventive nucleic acid(s) can comprises at least one intron and/or a polyA-sequence. According to another aspect of the present invention, the nucleic acid according to invention can be present in form of its complementary antisense-sequence Preferably, the nucleic acid is a synthetically produced nucleic acid.

The present invention also comprises nucleic acid sequences encoding the CARs receptors, which nucleic acid sequences are substantially the same as SEQ ID NOs. 1 to 10 as splice variants of CAR, their sequence complements or complements of said splice variants SEQ ID NOs. 1 to 10 encodes a human cDNA CAR receptor variant and is the preferred embodiment of the present invention.

Herein the "complement" refers to the complementary strand of the nucleic acid according to the invention, thus the strand that would hybridise to the nucleic acid according to the invention. In accordance with standard biological terminology all DNA sequences herein are however written in 5'-3' orientation, thus the if a complement is mentioned (see also figures) it is actually a "reverse" complement (as also stated in the figures). For simplification purposes they may however sometimes be referred to simply as "complements".

As used herein the term "a molecule having a nucleotide sequence substantially the same as SEQ ID NO y" (wherein "y" is the number of one of the protein-encoding nucleotide sequences listed in the Sequence Listing) means a nucleic acid encoding a protein "having an amino acid sequence substantially the same as SEQ ID NO y+1" (wherein "y+1" is the number of the amino acid sequence for which nucleotide sequence "y" codes) as defined above. This definition is intended to encompass natural allelic variations in the CARs sequence. Cloned nucleic acid provided by the present invention may encode CARs protein of any species of origin, including (but not limited to), for example, mouse, rat, rabbit, cat, dog, primate, and human.

### IDENTIFICATION OF VARIANTS AND HOMOLOGUES AS WELL AS USE OF PROBES:

Nucleic acid hybridisation probes provided by the invention are nucleic acids consisting essentially of the nucleotide sequences complementary to any sequence depicted in SEQ ID NOs. 1 to 10 or a part thereof and that are effective in nucleic acid hybridisation

Nucleic acid hybridisation probes provided by the invention are nucleic acids capable of detecting i.e. hybridising to the gene encoding the polypeptides according to SEQ ID NOs. 11 to 20.

Nucleic acid probes are useful for detecting CARs gene expression in cells and tissues using techniques well-known in the art, including, but not limited to, Northern blot hybridisation, in situ hybridisation, and Southern hybridisation to reverse transcriptase - polymerase chain reaction product DNAs. The probes provided by the present invention, including oligonucleotide probes derived therefrom, are also useful for Southern hybridisation of mammalian, preferably human, genomic DNA for screening for restriction fragment length polymorphism (RFLP) associated with certain genetic disorders. As used herein, the term complementary means a nucleic acid having a sequence that is sufficiently complementary in the Watson-Crick sense to a target nucleic acid to bind to the target under physiological conditions or experimental conditions those skilled in the art routinely use when employing probes.

It is understood in the art that a nucleic acid sequence will hybridise with a complementary nucleic acid sequence under high stringent conditions as defined herein, even though some mismatches may be present. Such closely matched, but not perfectly complementary sequences are also encompassed by the present invention. For example, differences may occur through genetic code degeneracy, or by naturally occurring or man made mutations and such mismatched sequences would still be encompassed by the present claimed invention.

Preferably, the nucleotide sequence of the nuclear receptor CARs (SEQ ID NOs. 1 to 10) and/or their complements can be used to derive oligonucleotide fragments (probes) of various length. If the probe is used to detect a human CARs sequence most preferably a complement of SEQ ID NOs. 1 to 10 is used. If the probe is supposed to detect a human, mouse or a rodent sequence probes complementary to SEQ ID NOs. 1 to 10, or their respective complements are preferred. Stretches of 17 to 30 nucleotides are used frequently but depending on the screening parameters longer sequences as 40, 50, 100, 150 up to the full length of the sequence may be used. Those probes can be synthesised chemically and are obtained readily from commercial oligonucleotide providers. Chemical synthesis has improved over the years and chemical synthesis of oligonucleotides as long as 100-200 bases is possible. The field might advance further to allow chemical synthesis of even longer fragments. Alternatively, probes can also be obtained by biochemical *de novo* synthesis of single stranded DNA. In this case the nucleotide sequence of the nuclear receptor CARs or its complement serve as a template and the corresponding complementary strand is synthesised. A variety of standard techniques such as nick translation or primer extension from specific primers or short random oligonucleotides can be used to synthesise the probe (Molecular Cloning: A Laboratory Manual (3 Volume Set) by Joseph Sambrook, David W. Russell, Joe Sambrook, 2100 pages 3rd edition (January 15, 2001; . Molecular cloning: a laboratory manual. Cold Spring Harbor Press, Cold Spring Harbor, 1989)). Nucleic acid reproduction technologies exemplified by the polymerase chain reaction (Saiki, R.K. et *al.* Primer-directed enzymatic amplification of DNA with a thermostable DNA polymerase. *Science* 239, 487-491 (1988)) are commonly applied to synthesise probes. In the case of techniques using specific primers the nucleic acid sequence of the nuclear receptor CARs or its complement is not only used as a template in the biochemical reaction but also to derive the specific primers which are needed to prime the reaction.

In some cases one might also consider to use the nucleic acid sequence of the cofactor or its complement as a template to synthesise an RNA probe. A promoter sequence for a DNA-dependent RNA polymerase has to be introduced at the 5'-end of sequence. As an example this can be done by cloning the sequence into a vector which carries the respective promoter sequence. It is also possible to introduce the needed sequence by synthesising a primer with the needed promoter in the form of a 5' "tail". The chemical synthesis of a RNA probe is another option.

Appropriate means are available to detect the event of a hybridisation. There is a wide variety of labels and detection systems, e.g. radioactive isotopes, fluorescent, or chemiluminescent molecules which can be linked to the probe. Furthermore, there are methods of introducing haptens which can be detected by antibodies or other ligands such as the avidin/biotin high affinity binding system.

Hybridisation can take place in solution or on solid phase or in combinations of the two, e.g. hybridisation in solution and subsequent capture of the hybridisation product onto a solid phase by immobilised antibodies or by ligand coated magnetic beads.

Hybridisation probes act by forming selectively duplex molecules with complementary stretches of a sequence of a gene or a cDNA. The selectivity of the process can be controlled by varying the conditions of hybridisation. To select sequences which are identical highly homologous to the sequence of interest stringent conditions for the hybridisation will be used, e.g. low salt in the range of 0.02 M to 0.15 M salt and/or high temperatures in the range from 50°C degrees centigrade to 70°C degrees centigrade. Stringency can be further improved by the addition of formamide to the hybridisation solution. The use of stringent conditions which means that only little mismatch or a complete match will lead to a hybridisation product would be used to isolate closely related members of the same gene family. Thus, as used herein stringent hybridisation conditions are those where between 0.02 M to 0.15 M salt and/or high temperatures in the range from 50°C degrees centigrade to 70°C degrees centigrade are applied.

The use of highly stringent conditions or conditions of "high stringency" means that only very little mismatch or a complete match which lead to a hybridisation product would be used to isolate very closely related members of the same gene family. Thus, as used herein highly stringent hybridisation conditions are those where between 0.02 - 0.3 M salt and 65°C degrees centigrade are applied for about 5 to 18 hours of hybridisation time and additionally, the sample filters are washed twice for about 15 minutes each at between 60°C - 65°C degrees centigrade, wherein the first washing fluid contains about 0.1 M salt (NaCl and/or Sodium Citrate) and the second contains only about 0.02 M salt (NaCl and/or Sodium Citrate). In a preferred embodiment the following conditions are considered to be highly stringent:

Hybridisation in a buffer containing 2 x SSC (0.03 M Sodium Citrate, 0.3 M NaCl at 65°C-68°C degrees centigrade for 12 hours, followed by a washing step for 15 minutes in 0.5 x SSC, 0.1% SDS, and a washing step for 15 minutes at 65°C degrees centigrade in 0.1 x SSC, 0.1% SDS.

Less stringent hybridization conditions, e.g. 0.15 M salt - 1 M salt and/or temperatures from 22°C degrees centigrade to 56°C degrees centigrade are applied in order to detect functionally equivalent genes in the same species or for orthologous sequences from other species.

Unspecific hybridization products are removed by washing the reaction products repeatedly in 2 x SSC solution and increasing the temperature.

### DEGENERATE PCR AND CLONING OF HOMOLOGUES

The nucleotide sequence of the nuclear receptor CARs or its complement can be used to design primers for a polymerase chain reaction. Due to the degeneracy of the genetic code the respective amino acid sequence is used to design oligonucleotides in which varying bases coding for the same amino acid are included. Numerous design rules for degenerate primers have been published (Compton et al, 1990). As in hybridization there are a number of factors known to vary the stringency of the PCR. The most important parameter is the annealing temperature. To allow annealing of primers with imperfect matches annealing temperatures are often much lower than the standard annealing temperature of 55°C, e.g. 35°C to 52°C degrees can be chosen. PCR reaction products can be cloned. Either the PCR product is cloned directly, with reagents and protocols from commercial manufacturers *(e.g.* from Invitrogen, San Diego, USA). Alternatively, restriction sites can be introduced intro the PCR product via a 5'-tail of the PCR primers and used for cloning. Primers for the amplification of the entire or partial pieces of the CARs gene or mRNA, or for reverse transcription may be designed making use of the sequences according to the invention, i.e. those depicted in the figures below.

### GENETIC VARIANTS

Fragments from the nucleotide sequence of the nuclear receptor CARs (SEQ ID NOs. 1 to 10) or their complements can be used to cover the whole sequence with overlapping sets of PCR primers. Also the genomic sequences may be used. These primers are used to produce PCR products using genomic DNA from a human diversity panel of healthy individuals or genomic DNA from individuals which are phenotypically conspicuous. Also the genomic sequences may be used, *i.e.* that of the human clone. The PCR products can be screened for polymorphisms, for example by denaturing gradient gel electrophoresis, binding to proteins detecting mismatches or cleaving heteroduplices or by denaturing high-performance liquid chromatography. Products which display mutations need to be sequenced to identify the nature of the mutation. Alternatively, PCR products can be sequenced directly omitting the mutation screening step to identify genetic polymorphisms. If genetic variants are identified and are associated with a discrete phenotype, these genetic variations can be included in diagnostic assays. The normal variation of the human population is of interest in designing screening assays as some variants might interact better or worse with a respective lead substance (a pharmacodynamic application). Polymorphisms or mutations which can be correlated to phenotypic outcome are a tool to extend the knowledge and the commercial applicability of the nucleotide sequence of the nuclear receptor CARs or its complement or their gene product, as variants might have a slightly different molecular behavior or desired properties. Disease-causing mutations or polymorphisms allow the replacement of this disease inducing gene copy with a wild-type copy by means of gene therapy approaches and/or the modulation of the activity of the gene product by drugs.

### PREPARATION OF POLYNUCLEOTIDES:

DNA which encodes receptor CARs may be obtained, in view of the instant disclosure, by chemical synthesis, by screening reverse transcripts of mRNA from appropriate cells or cell line cultures, by screening genomic libraries from appropriate cells, or by combinations of these procedures, as illustrated below.

Screening of mRNA or genomic DNA may be carried out with oligonucleotide probes generated from the CARs nucleotide sequences information provided herein.

Probes may be labelled with a detectable group such as a fluorescent group, a radioactive atom or a chemiluminescent group in accordance with known procedures and used in conventional hybridisation assays, as described in greater detail in the Examples below. Alternatively, the CARs nucleotide sequence may be obtained by use of the polymerase chain reaction (PCR) procedure, with the PCR oligonucleotide primers being produced from the CARs nucleotide sequences provided herein.

Upon purification or synthesis, the nucleic acid according to the invention may be labelled, e.g. for use as a probe.

As single and differential labelling agents and methods, any agents and methods which are known in the art can be used. For example, single and differential labels may consist of the group comprising enzymes such as β-galactosidase, alkaline phosphatase and peroxidase, enzyme substrates, coenzymes, dyes, chromophores, fluorescent, chemiluminescent and bio-luminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵I and ¹⁴C.

### EXPRESSION OF THE CARS PROTEIN/POLYPETIDE:

The nuclear receptor CARs nucleic acid or polypeptide may be synthesised in host cells transformed with a recombinant expression construct comprising a nucleic acid encoding the nuclear receptor CARs.

Such a recombinant expression construct can also be comprised of a vector that is a replicable DNA construct.

Amplification vectors do not require expression control domains. All that is needed is the ability to replicate in a host, usually conferred by an origin of replication, and a selection gene to facilitate recognition of transformants. See, Sambrook et al., Molecular Cloning: A Laboratory Manual (2nd Edition, Cold Spring Harbor Press, New York, 1989).

An expression vector comprises a polynucleotide operatively linked to a prokaryotic promoter. Alternatively, an expression vector is a polynucleotide operatively linked to an enhancer-promoter that is a eukaryotic promoter, and the expression vector further has a polyadenylation signal that is positioned 3' of the carboxy-terminal amino acid and within a transcriptional unit of the encoded polypeptide. A promoter is a region of a DNA molecule typically within about 500 nucleotide pairs in front of (upstream of) the point at which transcription begins (*i.e.,* a transcription start site). In general, a vector contains a replicon and control sequences which are derived from species compatible with the host cell. The vector ordinarily carries a replication site, as well as marking sequences which are capable of providing phenotypic selection in transformed cells.

Another type of discrete transcription regulatory sequence element is an enhancer. An enhancer provides specificity of time, location and expression level for a particular encoding region (e.g., gene). A major function of an enhancer is to increase the level of transcription of a coding sequence in a cell.

As used herein, the phrase "enhancer-promoter" means a composite unit that contains both enhancer and promoter elements. An enhancer-promoter is operatively linked to a coding sequence that encodes at least one gene product.

An enhancer-promoter used in a vector construct of the present invention may be any enhancer-promoter that drives expression in a prokaryotic or eukaryotic cell to be transformed/transfected.

A coding sequence of an expression vector is operatively linked to a transcription terminating region. RNA polymerase transcribes an encoding DNA sequence through a site where polyadenylation occurs.

An expression vector comprises a polynucleotide that encodes a nuclear receptor CARs polypeptide. Such a polynucleotide is meant to include a sequence of nucleotide bases encoding a nuclear receptor CARs polypeptide sufficient in length to distinguish said segment from a polynucleotide segment encoding a non- nuclear receptor CARs polypeptide.

A polypeptide of the invention may also encode biologically functional polypeptides or peptides which have variant amino acid sequences, such as with changes selected based on considerations such as the relative hydropathic score of the amino acids being exchanged.

These variant sequences are those isolated from natural sources or induced in the sequences disclosed herein using a mutagenic procedure such as site-directed mutagenesis.

Furthermore, an expression vector of the present invention may contain regulatory elements for optimised translation of the polypeptide in prokaryotic or eukaryotic systems. This sequences are operatively located around the transcription start site and are most likely similar to ribosome recognition sites like prokaryotic ribosome binding sites (RBS) or eukaryotic Kozak sequences as known in the art (Kozak M., Initiation of translation in prokaryotes and eukaryotes. *Gene* 234*,* 187-208 (1999).

An expression vector of the present invention is useful both as a means for preparing quantities of the nuclear receptor CARs polypeptide-encoding DNA itself, and as a means for preparing the encoded nuclear receptor CARs polypeptide and peptides. It is contemplated that where nuclear receptor CARs polypeptides of the invention are made by recombinant means, one may employ either prokaryotic or eukaryotic expression vectors as shuttle systems.

Where expression of recombinant nuclear receptor CARs polypeptides is desired and a eukaryotic host is contemplated, it is most desirable to employ a vector such as a plasmid, that incorporates a eukaryotic origin of replication. Additionally, for the purposes of expression in eukaryotic systems, one desires to position the nuclear receptor CARs encoding sequence or if desired parts thereof (SEQ ID NOs. 1 to 10) adjacent to and under the control of an effective eukaryotic promoter. To bring a coding sequence under control of a promoter, whether it is eukaryotic or prokaryotic, what is generally needed is to position the 5' end of the translation initiation site of the proper translational reading frame of the polypeptide between about 1 and about 2000 nucleotides 3' of or downstream with respect to the promoter chosen.

Furthermore, where eukaryotic expression is anticipated, one would typically desire to incorporate into the transcriptional unit which includes the nuclear receptor CARs polypeptide, an appropriate polyadenylation side.

The invention provides homogeneous compositions of mammalian nuclear receptor CARs polypeptide produced by transformed prokaryotic or eukaryotic cells as provided herein. Such homogeneous compositions are intended to be comprised of mammalian nuclear receptor CARs protein that comprises at least 90% of the protein in such homogenous composition. The invention also provides membrane preparation from cells expressing mammalian nuclear receptor CARs polypeptide as the result of transformation with a recombinant expression construct, as described here.

Within the scope of the present invention the terms recombinant protein or coding sequence both also include tagged versions of the proteins depicted in SEQ ID NOs. 11 to 20, and/or encoded by the nucleic acids according to the invention and fusion proteins of said proteins or parts thereof such as splice variants with any other recombinant protein. Tagged versions here means that small epitopes of 3-20 amino acids are added to the original protein by extending the coding sequence either at the 5 'or the 3' terminus leading to N-terminal or C-terminal extended proteins respectively, or that such small epitopes are included elsewhere in the protein. The same applies for fusion proteins where the added sequences are coding for longer proteins, varying between 2 and 100 kDa. Tags and fusion proteins are usually used to facilitate purification of recombinant proteins by specific antibodies or affinity matrices or to increase solubility of recombinant proteins within the expression host. Fusion proteins are also of major use as essential parts of yeast two hybrid screens for interaction partners of recombinant proteins.

Tags used in the scope of the present invention may include but are not limited to the following: EEF (alpha Tubulin), B-tag (QYPALT), E tag (GAPVPYPDPLEPR) c-myc Tag (EQKLISEEDL), Flag epitope (DYKDDDDK, HA tag (YPYDVPDYA), 6 or 10 x His Tag, HSV (QPELAPEDPED), Pk-Tag (GKPIPNPLLGLDST), protein C (EDQVDPRLIDGK), T7 (MASMTGGQQMG), VSV-G (YTDIEMNRLGK), Fusion proteines may include Thioredoxin, Glutathiontransferase (GST), Maltose binding Protein (MBP), Cellulose Binding protein (CBD), chitin binding protein, ubiquitin, the Fc part of Immunoglobulins, and the IgG binding domain of *Staphylococcus aureus* protein A. These examples of course are illustrative and not limiting and the standard amino acid one letter code was used above.

For expression of recombinant proteins in living cells or organisms, vector constructs harboring recombinant CARs nuclear receptor as set forth in SEQ ID NOs. 1 to 10 are transformed or transfected into appropriate host cells. Preferably, a recombinant host cell of the present invention is transfected with a polynucleotide of SEQ ID NOs. 1 to 10.

Means of transforming or transfecting cells with exogenous polynucleotide such as DNA molecules are well known in the art and include techniques such as calcium-phosphate- or DEAE-dextran-mediated transfection, protoplast fusion, electroporation, liposome mediated transfection, direct microinjection and virus infection (Sambrook et al., 1989).

The most frequently applied technique for transformation of prokaryotic cells is transformation of bacterial cells after treatment with calciumchloride to increase permeability (Dagert & Ehrlich, 1979), but a variety of other methods is also available for one skilled in the art.

The most widely used method for transfection of eukaryotic cells is transfection mediated by either calcium phosphate or DEAE-dextran. Although the mechanism remains obscure, it is believed that the transfected DNA enters the cytoplasm of the cell by endocytosis and is transported to the nucleus. Depending on the cell type, up to 90% of a population of cultured cells may be transfected at any one time. Because of its high efficiency, transfection mediated by calcium phosphate or DEAE-dextran is the method of choice for studies requiring transient expression of the foreign nucleic acid in large numbers of cells. Calcium phosphate-mediated transfection is also used to establish cell lines that integrate copies of the foreign DNA, which are usually arranged in head-to-tail tandem arrays into the host cell genome.

In the protoplast fusion method, protoplasts derived from bacteria carrying high numbers of copies of a plasmid of interest are mixed directly with cultured mammalian cells. After fusion of the cell membranes (usually with polyethylene glycol), the contents of the bacterium are delivered into the cytoplasm of the mammalian cells and the plasmid DNA is transported to the nucleus. Protoplast fusion is not as efficient as transfection for many of the cell lines that are commonly used for transient expression assays, but it is useful for cell lines in which endocytosis of DNA occurs inefficiently. Protoplast fusion frequently yields multiple copies of the plasmid DNA tandemly integrated into the host chromosome.

The application of brief, high-voltage electric pulses to a variety of mammalian and plant cells leads to the formation of nanometer sized pores in the plasma membrane. DNA is taken directly into the cell cytoplasm either through these pores or as a consequence of the redistribution of membrane components that accompanies closure of the pores. Electroporation may be extremely efficient and may be used both for transient expression of cloned genes and for establishment of cell lines that carry integrated copies of the gene of interest. Electroporation, in contrast to calcium phosphate-mediated transfection and protoplast fusion, frequently gives rise to cell lines that carry one, or at most a few, integrated copies of the foreign DNA.

Liposome transfection involves encapsulation of DNA and RNA within liposomes, followed by fusion of the liposomes with the cell membrane. The mechanism of how DNA is delivered into the cell is unclear but transfection efficiencies may be as high as 90%.

Direct microinjection of a DNA molecule into nuclei has the advantage of not exposing DNA to cellular compartments such as low-pH endosomes. Microinjection is therefore used primarily as a method to establish lines of cells that carry integrated copies of the DNA of interest.

The use of adenovirus as a vector for cell transfection is well known in the art. Adenovirus vector-mediated cell transfection has been reported for various cells (Stratford-Perricaudet et al., 1992).

A transfected cell may be prokaryotic or eukaryotic, transfection may be transient or stable. Where it is of interest to produce a full length human or mouse CARs protein, cultured mammalian mouse, or human cells are of particular interest.

In another aspect, the recombinant host cells of the present invention are prokaryotic host cells. In addition to prokaryotes, eukaryotic microbes, such as yeast may also be used illustrative examples for suitable cells and organisms for expression of recombinant proteins are belonging to but not limited to the following examples: Insect cells, such as Drosophila Sf21, SF9 cells or others, Expression strains of *Escherichia coli,* such as XL1 blue, BRL21, M15, *Saccharomyces cerevisiae, Schizosaccharomyces pombe, Hansenlua polymorpha and Pichia pastoris* strains, immortalised mammalian cell lines such as AtT-20, VERO and HeLa cells, Chinese hamster ovary (CHO) cell lines, and W138, BHK, COSM6, COS-7, 293 and MDCK cells, BHK-21 cells, Att 20HeLa cells, HeK 294, T47 D cells and others.

Expression of recombinant proteins within the scope of this invention can also be performed *in vitro.* This may occur by a two step procedure, thereby producing first mRNA by in vitro transcription of an apt polynucleotide construct followed by in vitro translation with convenient cellular extracts. These cellular extracts may be reticulocyte lysates but are not limited to this type. In vitro transcription may be performed by T7 or SP6 DNA polymerase or any other RNA polymerase which can recognise *per se* or with the help of accessory factors the promoter sequence contained in the recombinant DNA construct of choice. Alternatively one of the recently made available one step coupled transcription/translation systems may be used for in vitro translation of DNA coding for the proteins of this invention, e.g. from Roche Molecular Biochemicals. One illustrative but not limiting example for such a system is the TNT® T7 Quick System by Promega.

Expression of recombinant proteins in transfected cell may occur constitutively or upon induction. Procedures depend on the Cell/vector combination used and are well known in the art. In all cases, transfected cells are maintained for a period of time sufficient for expression of the recombinant CARs nuclear receptor protein. A suitable maintenance time depends strongly on the cell type and organism used and is easily ascertainable by one skilled in the art. Typically, maintenance time is from about 2 hours to about 14 days. For the same reasons and for sake of protein stability and solubility incubation temperatures during maintenance time may vary from 20°C to 42 °C.

Recombinant proteins are recovered or collected either from the transfected cells or the medium in which those cells are cultured. Recovery comprises cell disruption, isolation and purification of the recombinant protein. Isolation and purification techniques for polypeptides are well-known in the art and include such procedures as precipitation, filtration, chromatography, electrophoresis and the like.

In a preferred embodiment, purification includes but is not limited to affinity purification of tagged or non-tagged recombinant proteins. This is a well established robust technique easily adapted to any tagged protein by one skilled in the art. For affinity purification of tagged proteins, small molecules such as gluthathione, maltose or chitin, specific proteins such as the IgG binding domain of Staphylococcus aureus protein A, antibodies or specific chelates which bind with high affinity to the tag of the recombinant protein are employed. For affinity purification of non-tagged proteins specific monoclonal or polyclonal antibodies, which were raised against said protein, can be used. Alternatively immobilised specific interactors of said protein may be employed for affinity purification. Interactors include native or recombinant proteins as well as native or artificial specific low molecular weight ligands.

### CHEMICAL SYNTHESIS OF THE POLYPEPTIDE ACCORDING TO THE INVENTION:

Alternatively, the protein itself may be produced using chemical methods to synthesise any of the amino acid sequences according to the invention or that is encoded by the nucleotide sequences according to the invention (SEQ ID NOs. 1 to 10) and/or a portion thereof and/or splice variants thereof. For example, peptide synthesis can be performed using conventional Merrifield solid phase f-Moc or t-Boc chemistry or various solid-phase techniques (Roberge, J. Y. et al. (1995) Science 269: 202-204) and automated synthesis may be achieved, for example, using the ABI 431A Peptide Synthesizer (Perkin Elmer). The newly synthesised peptide(s) may be substantially purified by preparative high performance liquid chromatography (e.g., Creighton, T. (1983) Proteins, Structures and Molecular Principles, WH Freeman and Co., New York, N.Y.). The composition of the synthetic peptides may be confirmed by amino acid analysis or sequencing *(e.g.,* the Edman degradation procedure; Creighton, supra). Additionally, the amino acid sequences according to the invention, *i.e.* SEQ ID NOs. 11 to 20 or the sequences that are encoded by SEQ ID NOs. 11 to 20 or any part thereof, may be altered during direct synthesis and/or combined using chemical methods with sequences from other proteins, or any part thereof, to produce a variant polypeptide.

### SCREENING ASSAYS

The invention also concerns a method for screening for agents which are capable of activating the cellular function of the nuclear receptor CARs comprising the steps of contacting one or more candidate agents with a polypeptide according to the invention, removing unbound agent(s) and detecting whether the agent(s) interact with the polypeptide of the nuclear receptor.

The invention also concerns method for activating the cellular function of the nuclear receptor CARs, comprising the steps of contacting a cell with a binding agent of a polypeptide previously identified as outlined herein whereby the cellular function of CARs is activated.

Such a binding agent may be an antibody, RNA, an anti-sense oligonucleotide, a ribozyme or one of substances shown below or identified in a respective assay as disclosed herein.

In still a further embodiment, the present invention concerns a method for identifying new nuclear receptor inhibitory or stimulatory substances, which may be termed as "candidate substances". It is contemplated that this screening technique proves useful in the general identification of compounds that serve the purpose of inhibiting or stimulating nuclear receptor activity.

This also includes the use of heteromultimeric complexes of the nuclear receptor with other proteins.

Accordingly, in screening assays to identify pharmaceuticals agents which affect nuclear receptor activity, it is proposed that compounds isolated from natural sources, such as fungal extracts, plant extracts, bacterial extracts, higher eukaryotic cell extracts, or even extracts from animal sources, or marine, forest or soil samples, may be assayed for the presence of potentially useful pharmaceutical agents.

It will be understood that that the pharmaceutical agents to be screened could also be derived from chemical compositions or man-made compounds. The candidate substances can could also include monoclonal or polyclonal antibodies, peptides or proteins, such as those derived from recombinant DNA technology or by other means, including chemical peptide synthesis. The active compounds may include fragments or parts or derivatives of naturally-occurring compounds or may be only found as active combinations of known compounds which are otherwise inactive. We anticipate that such screens will in some cases lead to the isolation of agonists of nuclear receptors, in other cases to the isolation of antagonists. In other instances, substances will be identified that have mixed agonistic and antagonistic effects, or affect nuclear receptors in any other way. Assay systems that can be used are reporter gene assays, yeast-two-hybrid systems, ligand competitive assays (e.g. scintillation proximity assays), FRET-assays, fluorescence polarisation assays, and others.

### CELL BASED ASSAYS

To identify a candidate substance capable of influencing CARs nuclear receptor activity, one first obtains a recombinant cell line. One designs the cell line in such a way that the activity of the nuclear receptor leads to the expression of a protein which has an easily detectable phenotype (a reporter), such as luciferase, fluorescent proteins such as green or red fluorescent protein, beta-galactosidase, alpha-galactosidase, beta-lactamase, chloramphenicol-acetyl-transferase, beta-glucuronidase, or any protein which can be detected by a secondary reagent such as an antibody.

Methods for detecting proteins using antibodies, such as ELISA assays, are well known to those skilled in the art.

Here, the amount of reporter protein present reflects the transcriptional activity of the nuclear receptor. This recombinant cell line is then screened for the effect of substances on the expression of the reporters, thus measuring the effect of these substances on the activity of the nuclear receptor. These substances can be derived from natural sources, such as fungal extracts, plant extracts, bacterial extracts, higher eukaryotic cell extracts, or even extracts from animal sources, or marine, forest or soil samples, may be assayed for the presence of potentially useful pharmaceutical agents. It will be understood that that the pharmaceutical agents to be screened may be derived from chemical compositions or man-made compounds.

The candidate substances can also include monoclonal or polyclonal antibodies, peptides or proteins, such as those derived from recombinant DNA technology or by other means, including chemical peptide synthesis. The active compounds may include fragments or parts or derivatives of naturally-occurring compounds or may be only found as active combinations of known compounds which are otherwise inactive.

In general the assay comprises, contacting a suitable cell containing a reporter under the control of the CARs nuclear receptor with a test compound, monitoring said host cell for the expression of the reporter gene, wherein expression of the reporter reflects the transcriptional activity of the nuclear receptor CARs, and therefore reflects effects of the compound on the nuclear receptor.

In other embodiments of the invention assays are included where measuring the activity of a dimer of the nuclear receptor CARs and another protein. Further included are assays aiming at the identification of compounds which specifically influence only the monomeric, homodimeric or homomultimeric form of the nuclear receptor, or influencing only multimeric forms of the nuclear receptor. Such assays include measuring the effect of a compound on the nuclear receptor in the absence of a binding partner, and measuring the effect of a compound on the nuclear receptor in the presence of a binding partner. One skilled in the art will find numerous more assays which are equally covered by the invention.

A cell line where the activity of a nuclear receptor determines the expression of a reporter can be obtained by creating a fusion gene driving the expression of a fusion protein consisting of the ligand-binding domain of the CARs nuclear receptor fused to the DNA binding domain of a transcription factor with a known specificity for a given DNA sequence (the binding site). This DNA sequence (the binding site) can then be inserted in one or multiple copies before (5') to the promoter driving the expression of the reporter. Transcription factors useful for this approach include bacterial proteins, such as lexA, yeast proteins, such as Gal4, mammalian proteins such as NFkappaB or NFAT, the glucocorticoid receptor, the estrogen receptor, or plant proteins. The binding sites for these proteins can all be used in combination with the appropriate transcription factor to generate a useful reporter assay.

Another way to screen for inhibitors is to identify binding sites on DNA for the CARs nuclear receptor, and operatively link this binding site to a promoter operatively linked to a reporter gene. Included among others are binding sites for heterodimers of the CARs nuclear receptor with another protein.

Furthermore, transgenic animals described in the invention can be used to derive cell lines useful for cellular screening assays.

Cell lines useful for such an assay include many different kinds of cells, including prokaryotic, animal, fungal, plant and human cells. Yeast cells can be used in this assay, including *Saccharomyces cerevisiae* and *Schizosaccharomyces pombe* cells.

Another way to build cellular assays to measure the effect of compounds is the use of the yeast two hybrid system (see for example see, for example, U.S. Pat. No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; PCT Publication No. WO 94/10300, and U.S. Pat. No. 5,667,973), and or possible variants of the basic two hybrid system as discussed *e.g* in Vidal M, Legrain P, Nucleic Acids Res. 1999 Feb 15;27(4):919-29. Briefly, the two hybrid assay relies on reconstituting in vivo a functional transcriptional activator protein from two separate fusion proteins. In particular, the method makes use of chimeric genes which express hybrid proteins. To illustrate, a first hybrid gene comprises the coding sequence for a DNA-binding domain of a transcriptional activator fused in frame to the coding sequence for a TI polypeptide. The second hybrid protein encodes a transcriptional activation domain fused in frame to a sample gene from a cDNA library. If the bait and sample hybrid proteins are able to interact, e.g., form a TI-dependent complex, they bring into close proximity the two domains of the transcriptional activator. This proximity is sufficient to cause transcription of a reporter gene which is operably linked to a transcriptional regulatory site responsive to the transcriptional activator, and expression of the reporter gene can be detected and used to score for the interaction of the TI and sample proteins.

In such assays, one primarily measures the effect of a compound on a given interaction involving the CARs nuclear receptor and a binding protein. In a preferred embodiment of the invention systems using other hosts such as prokaryotes as *E. coli,* or eukaryotic mammalian cells are described.

Two hybrid systems using hybrid protein fusions with other proteins than transcription factors, including enzymes such as beta-galactosidase or *dihydrofolate reductase* may also be applied. These assays are useful both to monitor the effect of a compound, including peptides, proteins or nucleic acids on an interaction of a nuclear receptor with a given binding partner, as well as to identify novel proteins or nucleic acids interacting with the nuclear receptor.

Monitoring the influence of compounds on cells may be applied not only in basic drug screening, but also in clinical trials. In such clinical trials, the expression of a panel of genes may be used as a "read out" of a particular drug's therapeutic effect.

### CELL-FREE ASSAYS

Recombinant forms of the polypeptides according to SEQ ID NOs. 11 to 20 or as encoded by the nucleic acids according to the invention can be used in cell-free screening assays aiming at the isolation of compounds affecting the activity of nuclear receptors. In such an assay, the nuclear receptor polypeptide is brought into contact with a substance to test if the substance has an effect on the activity of the CARs receptor.

The detection of an interaction between an agent and a receptor may be accomplished through techniques well-known in the art. These techniques include but are not limited to centrifugation, chromatography, electrophoresis and spectroscopy. The use of isotopically labeled reagents in conjunction with these techniques or alone is also contemplated. Commonly used radioactive isotopes include ³H, ¹⁴C, ²²Na, ³²P, ³³P, ³⁵S, ⁴⁵Ca, ⁶⁰Co, ¹²⁵I, and ¹³¹I. Commonly used stable isotopes include ²H,¹³ C, ¹⁵N, ¹⁸O.

For example, if an agent binds to the receptor of the present invention, the binding may be detected by using radiolabeled agent or radiolabeled receptor. Briefly, if radiolabeled agent or radiolabeled receptor is utilized, the agent-receptor complex may be detected by liquid scintillation or by exposure to x-ray film or phosho-imaging devices.

One way to screen for substances affecting nuclear receptor activity is to measure the effect of the binding of nuclear receptors to ligands, such as cofactors, activators, repressors, DNA, RNA, proteins, antibodies, peptides or other substances, including chemical compounds known to affect receptor activity. Assays measuring the binding of a protein to a ligand are well known in the art, such as ELISA assays, FRET assays, bandshift assays, plasmonresonance based assays, scintilllation proximity assays, fluorescence polarization assays.

In one example, a mixture containing the CARs polypeptide, effector and candidate substance is allowed to incubate. The unbound effector is separable from any effector/receptor complex so formed. One then simply measures the amount of each (*e.g.*, versus a control to which no candidate substance has been added). This measurement may be made at various time points where velocity data is desired. From this, one determines the ability of the candidate substance to alter or modify the function of the receptor.

Numerous techniques are known for separating the effector from effector/receptor complex, and all such methods are intended to fall within the scope of the invention. This includes the use of thin layer chromatographic methods (TLC), HPLC, spectrophotometric, gas chromatographic/mass spectrophotometric or NMR analyses. Another method of separation is to immobilise one of the binding partners on a solid support, and to wash away any unbound material. It is contemplated that any such technique may be employed so long as it is capable of differentiating between the effector and complex, and may be used to determine enzymatic function such as by identifying or quantifying the substrate and product.

A screening assay provides a CARs receptor under conditions suitable for the binding of an agent to the CARs receptor. These conditions include but are not limited to pH, temperature, tonicity, the presence of relevant cofactors, and relevant modifications to the polypeptide such as glycosylation or lipidation. It is contemplated that the receptor can be expressed and utilised in a prokaryotic or eukaryotic cell. The host cell expressing the CARs receptor can be used whole or the receptor can be isolated from the host cell. The CARs receptor can be membrane bound in the membrane of the host cell or it can be free in the cytosol of the host cell. The host cell can also be fractionated into sub-cellular fractions where the receptor can be found. For example, cells expressing the receptor can be fractionated into the nuclei, the endoplasmic reticulum, vesicles, or the membrane surfaces of the cell.

pH is preferably from about a value of 6.0 to a value of about 8.0, more preferably from about a value of about 6.8 to a value of about 7.8, and most preferably, about 7.4. In a preferred embodiment, temperature is from about 20°C degrees to about 50°C degrees more preferably, from about 30°C degrees to about 40°C degrees and even more preferably about 37°C degrees. Osmolality is preferably from about 5 milliosmols per liter (mosm/L) to about 400 mosm/l, and more preferably, from about 200 milliosmols per liter to about 400 mosm/l and, even more preferably from about 290 mosm/L to about 310 mosm/L. The presence of cofactors can be required for the proper functioning of the CARs receptor. Typical cofactors include sodium, potassium, calcium, magnesium, and chloride. In addition, small, non-peptide molecules, known as prosthetic groups may also be required. Other biological conditions needed for receptor function are well-known in the art.

It is well-known in the art that proteins can be reconstituted in artificial membranes, vesicles or liposomes. (Danboldt et al.,1990). The present invention contemplates that the receptor can be incorporated into artificial membranes, vesicles or liposomes. The reconstituted receptor can be utilised in screening assays.

It is further contemplated that a receptor of the present invention can be coupled to a solid support, e.g., to agarose beads, polyacrylamide beads, polyacrylic, sepharose beads or other solid matrices capable of being coupled to polypeptides. Well-known coupling agents include cyanogen bromide (CNBr), carbonyldiimidazole, tosyl chloride, diaminopimelimidate, and glutaraldehyde.

In a typical screening assay for identifying candidate substances, one employs the same recombinant expression host as the starting source for obtaining the receptor polypeptide, generally prepared in the form of a crude homogenate. Recombinant cells expressing the receptor are washed and homogenised to prepare a crude polypeptide homogenate in a desirable buffer such as disclosed herein. In a typical assay, an amount of polypeptide from the cell homogenate, is placed into a small volume of an appropriate assay buffer at an appropriate pH. Candidate substances, such as agonists and antagonists, are added to the admixture in convenient concentrations and the interaction between the candidate substance and the receptor polypeptide is monitored.

Where one uses an appropriate known substrate for the CARs receptor, one can, in the foregoing manner, obtain a baseline activity for the recombinantly produced CARs receptor. Then, to test for inhibitors or modifiers of the receptor function, one can incorporate into the admixture a candidate substance whose effect on the CARs receptor is unknown. By comparing reactions which are carried out in the presence or absence of the candidate substance, one can then obtain information regarding the effect of the candidate substance on the normal function of the receptor.

Accordingly, this aspect of the present invention will provide those of skill in the art with methodology that allows for the identification of candidate substances having the ability to modify the action of nuclear receptor polypeptides in one or more manners.

Additionally, screening assays for the testing of candidate substances are designed to allow the determination of structure-activity relationships of agonists or antagonists with the receptors, e.g., comparisons of binding between naturally-occurring hormones or other substances capable of interacting with or otherwise modulating the receptor; or comparison of the activity caused by the binding of such molecules to the receptor.

In certain aspects, the polypeptides of the invention are crystallised in order to carry out x-ray crystallographic studies as a means of evaluating interactions with candidate substances or other molecules with the nuclear receptor polypeptide. For instance, the purified recombinant polypeptides of the invention, when crystallised in a suitable form, are amenable to detection of intra-molecular interactions by x-ray crystallography. In another aspect, the structure of the polypeptides can be determined using nuclear magnetic resonance.

### PHARMACEUTICAL COMPOSITION:

This invention provides a pharmaceutical composition comprising an effective amount of a agonist or antagonist drug identified by the method described herein and a pharmaceutically acceptable carrier. Such drugs and carrier can be administered by various routes, for example oral, subcutaneous, intramuscular, intravenous or intracerebral. The preferred route of administration would be oral at daily doses of about 0.01 -100 mg/kg.

This invention provides a method of treating metabolic disorders, immunological indications, hormonal dysfunctions, neurosystemic diseases wherein the abnormality is improved by reducing the activity of CARs receptor or blocking the binding of ligands to a CARs receptor, which method comprises administering an effective amount of the antagonist-containing pharmaceutical composition described above to suppress the subject's appetite. Similarly, the invention also provides methods for treating diseases and conditions resulting from metabolic disorders, toxification, immunological indications, hormonal dysfunctions, neurosystemic diseases, apoptosis, and/or inflammation which method comprises administering an effective amount of an agonist-containing pharmaceutical composition described above.

### TRANSFORMATION OF CELLS AND DRUG SCREENING :

The recombinant expression constructs of the present invention are useful in molecular biology to transform cells which do not ordinarily express CARs to thereafter express this receptor.

Such cells are useful as intermediates for making cellular preparations useful for receptor binding assays, which are in turn useful for drug screening. Drugs identified from such receptor-assays can be used for the treatment of metabolic disorders, toxification(s), apoptosis, immunological indications, hormonal dysfunctions, and/or neurosystemic diseases.

The recombinant expression constructs of the present invention are also useful in gene therapy. Cloned genes of the present invention, or fragments thereof, may also be used in gene therapy carried out by homologous recombination or site-directed mutagenesis. See generally Thomas & Capecchi, Cell 51, 503-512 (1987); Bertling, Bioscience Reports 7, 107-112 (1987); Smithies et al., Nature 317, 230-234 (1985).

Oligonucleotides of the present invention are useful as diagnostic tools for probing CARs expression in tissues. For example, tissues are probed *in situ* with oligonucleotide probes carrying detectable groups by conventional autoradiographic techniques, as explained in greater detail in the Examples below, to investigate native expression of this receptor or pathological conditions relating thereto. Further, chromosomes can be probed to investigate the presence or absence of the CARs, and potential pathological conditions related thereto, as also illustrated by the Examples below. Probes according to the invention should generally be at least about 15 nucleotides in length to prevent binding to random sequences, but, under the appropriate circumstances may be smaller (see above for details on hybridization).

### ANTIBODIES AGAINST THE CARS NUCLEAR RECEPTOR PROTEIN OR POLYPEPTIDE

Another aspect of the invention includes an antibody specifically reactive with the protein or any part of the protein according to the invention (SEQ ID NO.?) and or a polypeptide encoded by the nucleotide sequence of the nuclear receptor CARs (The term "antibody" refers to intact molecules as well as fragments thereof, such as Fa, F(ab).sub.2, and Fv, which are capable of binding the epitopic determinant.) By using immunogens derived from the polypeptide according to the invention (SEQ ID NO. 2) and/or encoded by the nucleic acids according to the invention, anti-protein/anti-peptide antisera or monoclonal antibodies can be made by standard protocols (E. Howell & D. Lane. Antibodies: A Laboratory Manual. *Cold Spring Harbor Laboratory* (1988)).

A polyclonal antibody is prepared by immunizing a mammal, such as a mouse, a hamster or rabbit with an immunogenic form of the polypeptide, i.e. the human CARs polypeptide of the present invention, and collecting antisera from that immunized animal. Because of the relatively large blood volume of rabbits, a rabbit is a preferred choice for production of polyclonal antibodies.

As an immunizing antigen, fusion proteins, intact polypeptides or fragments containing small peptides of interest can be used. They can be derived by expression from a cDNA transfected in a host cell with subsequent recovering of the protein/peptide or peptides can be synthesized chemically *(e.g.* oligopeptides with 10-15 residues in length). Important tools for monitoring the function of the gene according to the present invention, *i.e.* encoded by a sequence according to SEQ ID NOs. 1 to 10 (or portions thereof or splice variants thereof) are antibodies against various domains of the protein according to the invention. Various Oligopeptides from the N- and C-terminal sequences and the DBD/hinge region of the protein can be used as antigens.

A given polypeptide or polynucleotide may vary in its immunogenicity. It is often necessary to couple the immunogen (e.g. the polypeptide) with a carrier. Commonly used carriers that are chemically coupled to peptides include bovine serum albumin (BSA) and keyhole limpet hemocyanin (KLH). The coupled peptide is then used to immunise the animal in the presence of an adjuvant, a non-specific stimulator of the immune response in order to enhance immunogenicity. The production of polyclonal antibodies is monitored by detection of antibody titers in plasma or serum at various time points following immunisation. Standard ELISA or other immunoassays can be used with the immunogen as antigen to assess the levels of antibodies. When a desired level of immunogenicity is obtained, the immunised animal may be bled and the serum isolated, stored and purified.

To produce monoclonal antibodies, antibody-producing cells (e.g. spleen cells) from an immunised animal (preferably mouse or rat) are fused by standard somatic cell fusion procedures with immortalizing cells such as myeloma cells to yield hybridoma cells. Where the immunized animal is a mouse, a preferred myeloma cell is the murine NS-1 myeloma cell. Such techniques are well known in the art, and include, for example, the hybridoma technique (originally developed by Kohler & Milstein. *Nature* 256: 495-497 (1975)), the human B cell hybridoma technique (Kozbar *et al. Immunology Today* 4:72 (1983)), and the EBV-hybridoma technique to produce human monoclonal antibodies (Cole *et al.* Monoclonal Antibodies and Cancer Therapy. *Alan R. Liss, Inc.* pp. 77-96 (1985)).

The fused spleen/myeloma cells are cultured in a selective medium to select fused spleen/myeloma cells from the parental cells. Fused cells are separated from the mixture of non-fused parental cells, for example, by the addition of agents that block the *de novo* synthesis of nucleotides in the tissue culture media. This culturing provides a population of hybridomas from which specific hybridomas are selected. Typically, selection of hybridomas is performed by culturing the cells by single-clone dilution in microtiter plates, followed by testing the individual clonal supernatants for reactivity with an antigen-polypeptide. The selected clones may then be propagated indefinitely to provide the monoclonal antibody in convenient quantity.

The creation of antibodies which specifically bind the polypeptide according to the invention (SEQ ID NOs. 11 to 20) and/or encoded by the nucleotide sequence of the nuclear receptor CARs or its complement (SEQ ID NOs. 1 to 10) provides an important utility in immunolocalization studies, and may play an important role in the diagnosis and treatment of receptor disorders. The antibodies may be employed to identify tissues, organs, and cells which express or the nuclear receptor CARs. Antibodies can be used diagnostically in immunoprecipitation and immuno-blotting to detect and evaluate nuclear receptor CARs protein levels in tissue or from cells in bodily fluid as part of a clinical testing procedure.

Monoclonal antibodies provided by the present invention are also produced by recombinant genetic methods well known to those of skill in the art, and the present invention encompasses antibodies made by such methods that are immunologically reactive with an epitope of a mammalian nuclear CARs receptor protein or peptide.

The present invention encompasses fragments of the antibody that are immunologically reactive with an epitope of a mammalian nuclear CARs receptor protein or peptide. Such fragments are produced by any number of methods, including but not limited to proteolytic cleavage, chemical synthesis or preparation of such fragments by means of genetic engineering technology. The present invention also encompasses single-chain antibodies that are immunologically reactive with an epitope of a mammalian nuclear CARs receptor protein or peptide made by methods known to those of skilled in the art.

### CHIMERIC ANTIBODIES AND OTHER TYPES OF ANTIBODIES:

The invention also includes chimeric antibodies, comprised of light chain and heavy chain peptides immunologically reactive to an epitope that is a mammalian nuclear CARs receptor protein or peptide. The chimeric antibodies embodied in the present invention include those that are derived from naturally occurring antibodies as well as chimeric antibodies made by means of genetic engineering technology well known to those of skill in the art.

Also included are methods for the generation of antibodies against CARs which rely on the use of phage display systems and related systems, such as described in Hoogenboom HR, de Bruine AP, Hufton SE, Hoet RM, Arends JW, Roovers RC, Immunotechnology 1998 Jun;4(1):1-20, and references therein.

### EPITOPES OF THE CARS NUCLEAR RECEPTOR

The present invention also encompasses an epitope of a mammalian nuclear CARs receptor protein or peptide that is comprised of sequences and/or a conformation of sequences present in the mammalian nuclear CARs receptor protein or peptide molecule. This epitope may be naturally occurring, or may be the result of proteolytic cleavage of the mammalian nuclear CARs receptor protein or peptide molecule and isolation of an epitope-containing peptide or may be obtained by synthesis of an epitope-containing peptide using method of genetic engineering technology and synthesized by genetically engineered prokaryotic or eukaryotic cells.

### ANTISENSE OLIGONUCLEOTIDES AGAINST CARS

Antisense oligonucleotides are short single stranded DNA or RNA molecules which may be used to block the availability of the CARs receptor messenger. Synthetic derivatives of ribonucleotides or deoxyribonucleotides and/or PNAs (see above) are equally possible.

The sequence of an antisense oligonucleotide is at least partially complementary to the sequence (or the gene) of interest. The complementarity of the sequence is in any case high enough to enable the antisense oligonucleotide to bind to the nucleic acid according to the invention or parts thereof. Many examples exist in which the binding of oligonucleotides to the target sequence interfere with the biological function of the targeted sequence (Brysch W, Schlingensiepen KH, Design and application of antisense oligonucleotides in cell culture, in vivo, and as therapeutic agents, Cell Mol Neurobiol 1994 Oct;14(5):557-68; Wagner RW, Gene inhibition using antisense oligodeoxynucleotides, Nature 1994 Nov 24;372(6504):333-5 or Brysch W, Magal E, Louis JC, Kunst M, Klinger I, Schlingensiepen R, Schlingensiepen KH Inhibition of p185c-erbB-2 proto-oncogene expression by antisense oligodeoxynucleotides down-regulates p185-associated tyrosine-kinase activity and strongly inhibits mammary tumor-cell proliferation, Cancer Gene Ther 1994 Jun;1(2):99-105 or Monia BP, Johnston JF, Ecker DJ, Zounes MA, Lima WF, Freier SM Selective inhibition of mutant Ha-ras mRNA expression by antisense oligonucleotides, J Biol Chem 1992 Oct 5;267(28):19954-62 or Bertram J, Palfner K, Killian M, Brysch W, Schlingensiepen KH, Hiddemann W, Kneba M, Reversal of multiple drug resistance in vitro by phosphorothioate oligonucleotides and ribozymes, Anticancer Drugs 1995 Feb;6(1):124-34)

This interference occurs in most instances at the level of translation, i.e. through the inhibition of the translational machinery by oligonucleotides that bind to mRNA, however, two other mechanisms of interference with a given gene's function by oligonucleotides can also be envisioned, (i) the functional interference with the transcription of a gene through formation of a triple helix at the level of genomic DNA and the interference of oligonucleotides with the function of RNA molecules that are executing at least part of their biological function in the untranslated form (Kochetkova M, Shannon MF, Triplex-forming oligonucleotides and their use in the analysis of gene transcription. Methods Mol Biol 2000;130:189-201 Rainer B. Lanz1, Neil J. McKenna1, Sergio A. Onate1, Urs Albrecht2, Jiemin Wong1, Sophia Y. Tsail, Ming-Jer Tsai1, and Bert W. O'Malley A Steroid Receptor Coactivator, SRA, Functions as an RNA and Is Present in an SRC-1 Complex Cell, Vol. 97, 17-27, April, 1999).

Antisense oligonucleotides can be conjugated to different other molecules in order to deliver them to the cell or tissue expressing CARs. For instance the antisense oligonucleotide can be conjugated to a carrier protein (*e.g.* ferritin) in order to direct the oligonucleotide towards the desired target tissue, *i.e.* in case of ferritin predominantly to the liver.

Antisense expression constructs are expression vector systems that allow the expression-either inducible or uninducible - of a complementary sequence to the CARs sequences according to the invention. The potential possibility of such an approach has been demonstrated in many different model systems (von Ruden T, Gilboa E, Inhibition of human T-cell leukemia virus type I replication in primary human T cells that express antisense RNA, J Virol 1989 Feb;63(2):677-82; Nemir M, Bhattacharyya D, Li X, Singh K, Mukherjee AB, Mukherjee BB, Targeted inhibition of osteopontin expression in the mammary gland causes abnormal morphogenesis and lactation deficiency, J Biol Chem 2000 Jan 14;275(2):969-76; Ma L, Gauville C, Berthois Y, Millot G, Johnson GR, Calvo F Antisense expression for amphiregulin suppresses tumorigenicity of a transformed human breast epithelial cell line, Oncogene 1999 Nov 11;18(47):6513-20; Refolo LM, Eckman C, Prada CM, Yager D, Sambamurti K, Mehta N, Hardy J, Younkin SG, Antisense-induced reduction of presenilin 1 expression selectively increases the production of amyloid beta42 in transfected cells, J Neurochem 1999 Dec;73(6):2383-8; Buckley NJ, Abogadie FC, Brown DA, Dayrell M, Caulfield MP, Delmas P, Haley JE, Use of antisense expression plasmids to attenuate G-protein expression in primary neurons, Methods Enzymol 2000;314:136-48).

According to the invention an antisense expression construct can be constructed with virtually any expression vector capable of fulfilling at least the basic requirements known to those skilled in the art.

In one embodiment of the invention retroviral expression systems or tissue specific gene expression systems are preferred.

Current standard technologies for delivering antisense constructs are performed through a conjugation of constructs with liposomes and related, complex-forming compounds, which are transferred via electroporation techniques or via particle-mediated "gene gun" technologies into the cell. Other techniques may be envisioned by one skilled in the art.

Microinjection still plays a major role in most gene transfer techniques for the generation of germ-line mutants expressing foreign DNA (including antisense RNA constructs) and is preferred embodiment of the present invention.

### RIBOZYMES DIRECTED AGAINST CARS

Ribozymes are either RNA molecules (Gibson SA, Pellenz C, Hutchison RE, Davey FR, Shillitoe EJ, Induction of apoptosis in oral cancer cells by an anti-bcl-2 ribozyme delivered by an adenovirus vector, Clin Cancer Res 2000 Jan;6(1):213-22; Folini M, Colella G, Villa R, Lualdi S, Daidone MG, Zaffaroni N, Inhibition of Telomerase Activity by a Hammerhead Ribozyme Targeting the RNA Component of Telomerase in Human Melanoma Cells, J Invest Dermatol 2000 Feb;114(2):259-267; Halatsch ME, Schmidt U, Botefur IC, Holland JF, Ohnuma T, Marked inhibition of glioblastoma target cell tumorigenicity in vitro by retrovirus-mediated transfer of a hairpin ribozyme against deletion-mutant epidermal growth factor receptor messenger RNA, J Neurosurg 2000 Feb;92(2):297-305; Ohmichi T, Kool ET, The virtues of self-binding: high sequence specificity for RNA cleavage by self-processed hammerhead ribozymes, Nucleic Acids Res 2000 Feb 1;28(3):776-783) or DNA molecules (Li J, Zheng W, Kwon AH, Lu Y, In vitro selection and characterization of a highly efficient Zn(II)-dependent RNA-cleaving deoxyribozyme; Nucleic Acids Res 2000 Jan 15;28(2):481-488) that have catalytic activity. The catalytic activity located in one part of the RNA (or DNA) molecule can be "targeted" to a specific sequence of interest by fusing the enzymatically active RNA molecule sequence with a short stretch of RNA (or DNA) sequence that is complementary to the CARs transcript. Such a construct will, when introduced into a cell either physically or via gene transfer of a ribozyme expression construct find the CARs sequence (our sequence of interest) and bind via its sequence-specific part to said sequence. The catalytic activity attached to the construct, usually associated with a special nucleic acid structure (people distinguish so called "hammerhead" structures and "hairpin" structures), will then cleave the targeted RNA. The targeted mRNA will be destroyed and cannot be translated efficiently, thus the protein encoded by the mRNA derived from CARs will not be expressed or at least will be expressed at significantly reduced amounts.

In a preferred embodiment the invention covers inducible ribozyme constructs (Koizumi M, Soukup GA, Kerr JN, Breaker RR, Allosteric selection of ribozymes that respond to the second messengers cGMP and cAMP, Nat Struct Biol 1999 Nov;6(11):1062-1071).

In a further preferred embodiment the invention concerns the use of "bivalent" ribozymes (multimers of catalytically active nucleic acids) as described in (Hamada M, Kuwabara T, Warashina M, Nakayama A, Taira K, Specificity of novel allosterically trans- and cis-activated connected maxizymes that are designed to suppress BCR-ABL expression FEBS Lett 1999 Nov 12;461(1-2):77-85).

### TRANSGENIC ANIMALS CARRYING THE CARS NUCLEAR RECEPTOR

Also provided by the present invention are non-human transgenic animals grown from germ cells transformed with the CARs nucleic acid sequence according to the invention and that express the CARs receptor according to the invention and offspring and descendants thereof. Also provided are transgenic non-human mammals comprising a homologous recombination knockout of the native CARs receptor, as well as transgenic non-human mammals grown from germ cells transformed with nucleic acid antisense to the CARs nucleic acid of the invention and offspring and descendants thereof. Further included as part of the present invention are transgenic animals which the native CARs receptor has been replaced with the human homologue. Of course, offspring and descendants of all of the foregoing transgenic animals are also encompassed by the invention.

Transgenic animals according to the invention can be made using well known techniques with the nucleic acids disclosed herein. E.g., Leder et al., U.S. Patent Nos.4,736,866 and 5,175,383; Hogan et al., Manipulating the Mouse Embryo, A Laboratory Manual (Cold Spring Harbor Laboratory (1986)); Capecchi, Science 244, 1288 (1989); Zimmer and Gruss, Nature 338, 150 (1989); Kuhn et al., Science 269, 1427 (1995); Katsuki et al., Science 241, 593 (1988); Hasty et al., Nature 350, 243 (1991); Stacey et al., Mol. Cell Biol. 14, 1009 (1994); Hanks et al., Science 269, 679 (1995); and Marx, Science 269, 636 (1995). Such transgenic animals are useful for screening for and determining the physiological effects of CARs receptor agonists and antagonist.

Consequently, such transgenic animals are useful for developing drugs to regulate physiological activities in which CARs participates.

The following examples are provided for illustrative purposes only and are not intended, nor should they be construed, as limiting the invention in any manner.

### MODELLING OF THE STRUCTURE OF CARS

The novel nuclear receptor sequences disclosed herein may be used for various in silico, i.e. computer analyses. Such analyses may be for example nuclear receptor specific sequence alignments which permit the identification of domains and even new receptors. The novel domain sequences disclosed herein may be used in order to create domain specific hidden markov models (hmms) or simply as search sequences.

In a preferred embodiment this similarity search tool is the BLAST algorithm. (Altschul, Stephen F., Warren Gish, Webb Miller, Eugene W. Myers, and David J. Lipman (1990). Basic local alignment search tool. J. Mol. Biol. 215:403-10 and the sequence used is one of those disclosed herein.

Another search tool that may be used is FASTA (W. R. Pearson and D. J. Lipman (1988), "Improved Tools for Biological Sequence Analysis", PNAS 85:2444- 2448, and W. R. Pearson (1990) "Rapid and Sensitive Sequence Comparison with FASTP and FASTA" Methods in Enzymology 183:63-98).

The invention is not limited to one particular type of search tool. In one embodiment of the invention search tools are used that do not search by sequence similarity but by applying sequence profiles such as a profile generated when applying the Profile Hidden Markov Model.

Profile Hidden Markov Models also called "Hidden Markov Models", here abbreviated as HMM, are statistical models representing the consensus of the primary structure of a sequence family. The profiles use scores specific of the position of amino acids (or nucleotides) and position specific scores for the opening or the expansion of an insertion or deletion. Methods for the creation of profiles, starting from multiple alignments, have been introduced by Taylor (1986), Gribskov et al. (1987), Barton (1990) and Heinikoff (1996).

HMMs provide an utterly probabilistic description of profiles, *i.e.* Bayes' theory rules the positioning of all probability (evaluation) parameters (compare Krogh et al. 1994, Eddy 1996 und Eddy 1998). The central idea behind this is that a HMM is a finite model describing the probability distribution of an infinite number of possible sequences. The HMM consists of a number of states corresponding with the columns of a multiple alignment as it is usually depicted. Each state emits symbols (remainders) corresponding with the probability of the symbol emission (specific of the respective state), and the states are linked with each other by probabilities of the changing of states. Starting from one specific state, a succession of states is generated by changing from one state to the other in accordance with the probability of the changing of states, until a final state has been reached. Each state then emits symbols according to the probability distribution of emissions specific of this state, creating an observable sequence of symbols.

The attribute "hidden" has been derived from the fact that the underlying sequence of states cannot be observed. Only the sequence of symbols is visible. An assessment of the probabilities of changing of states and of emissions (the training of the model) is achieved by dynamic programming algorithms implemented in the HMMER package.

The sequences according to the invention may be aligned with other nuclear receptor sequences in order to create a multiple sequence alignment which is used as the basis for the creation of a HMM.

If an existing HMM and a sequence are given, the probability that the HMM could generate the sequence in question, can be calculated. The HMMER package provides a numerical quantity (the Score) in proportion to this probability, *i.e.* the information content of the sequence indicated as bits, measured according to the HMM.

See also Barton, G.J. (1990): Protein multiple alignment and flexible pattern matching, Methods Ezymol. 183: 403-427, Eddy, S.R. (1996): Hidden markov models. Curr. Opin. Strct. Biol. 6: 361-365, Eddy, S.R. (1998): Profile hidden markov models.Bioinformatics. 14: 755-763, Gribskov, M. McLachlan, A.D. und Eisenberg D. (1987): Profile analysis: Detection of distantly related proteins. Proc. Natl. Acad. Sci. USA. 84: 4355-5358, Heinikoff, S. (1996): Scores for sequence searches and alignment, Curr. Opin. Strct. Biol. 6: 353-360, Krogh, A., Brown, M., Mian, I.S., Sjolander, K. und Haussler, D. (1994): Hidden markov models in computational biology: Applications to protein modelling. J. Mol. Biol. 235: 1501-1531, Taylor, W.R. (1986): Identification of protein sequence homology by consensus template alignment. J. Mol. Biol. 188: 233-258.

In general the sequence are selected such that a query using a search sequence returns a result consisting of sequences which are at least to a certain degree similar to the query sequence.

In one embodiment of the invention amino acid sequences of the present invention are used to model the three-dimensional structure of the protein. Initially, this involves the comparison of the protein sequence with the sequence of related proteins where the structure is known, such as the human PPARγ ligand-binding domain (Nolte RT, Wisely GB, Westin S, Cobb JE, Lambert MH, Kurokawa R, Rosenfeld MG, Willson TM, Glass CK, Milburn MV,Nature 1998 Sep 10;395(6698):137-43). The three-dimensionale structure can then be modelled using computer programs. From the three-dimensional structure, binding sites of potential inhibitors or activators can be predicted. It can further be predicted which kinds of molecule might bind there. The predicted substances can then be screened to test their effect on nuclear receptor activity.

The present invention shall now be further described in the following example with respect to the accompanying figure and sequence protocol. In the figure and sequence protocol,

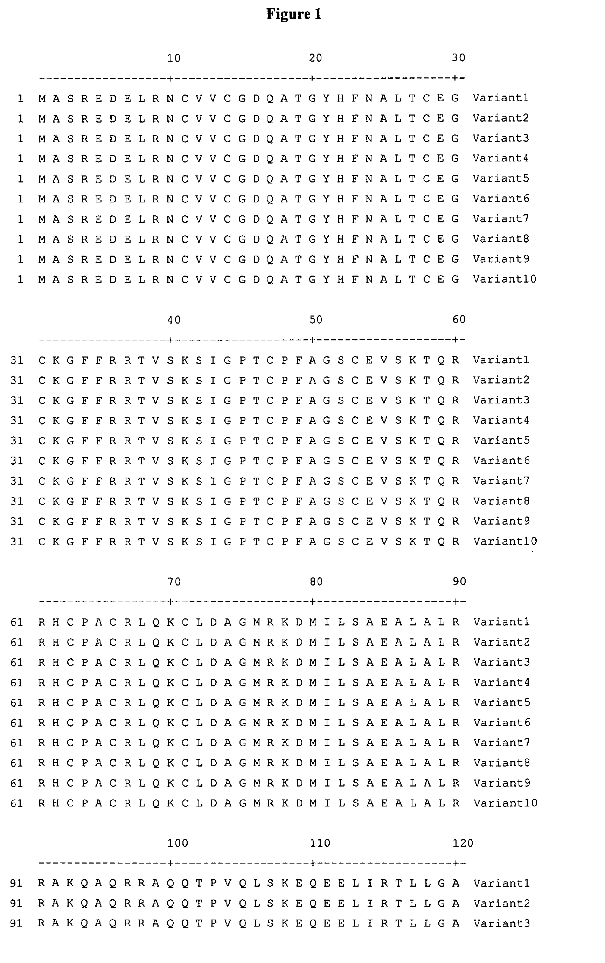

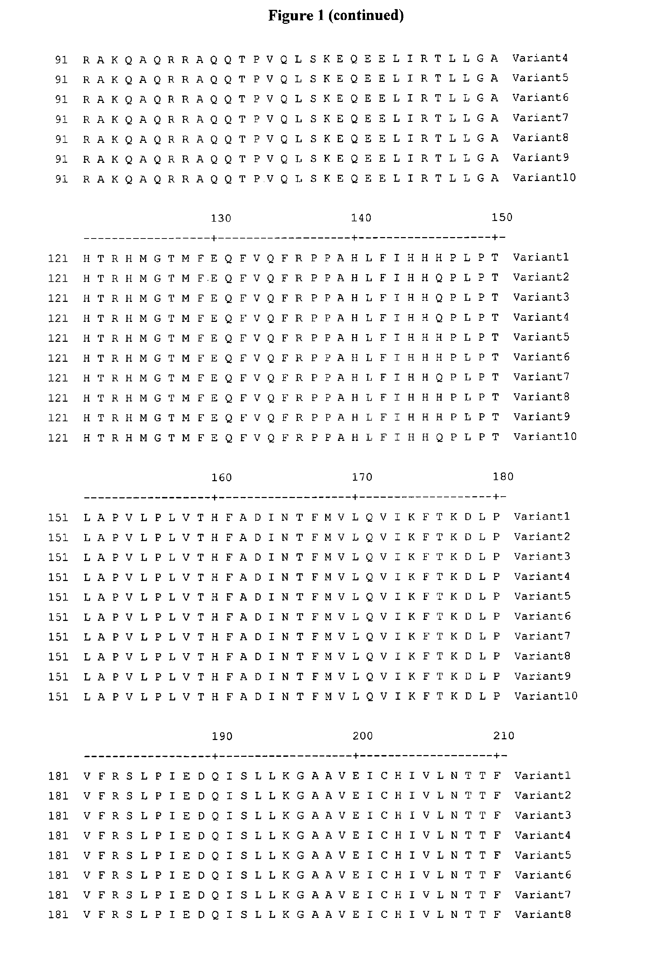

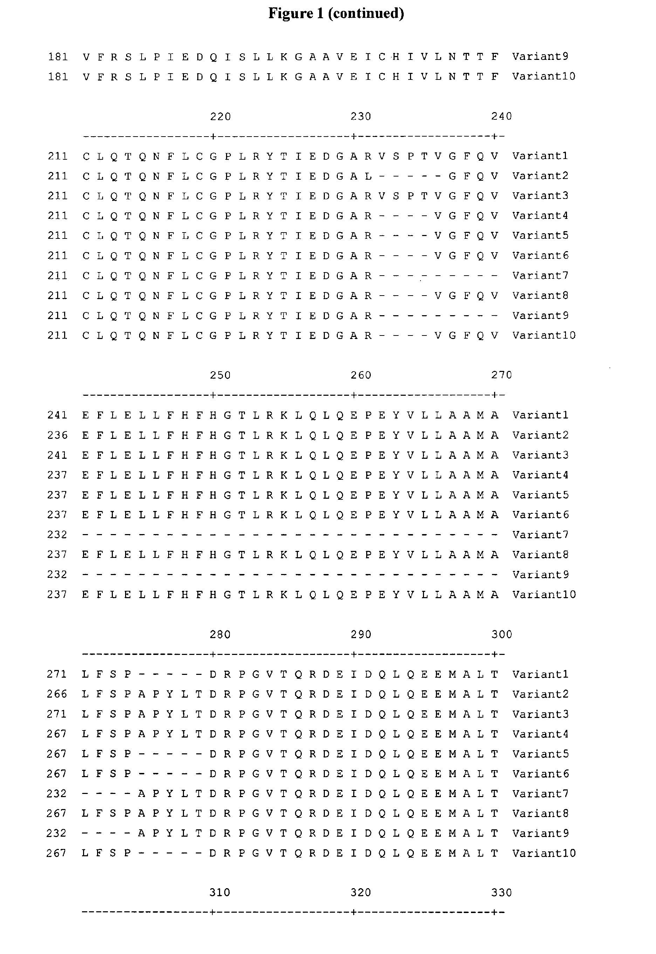

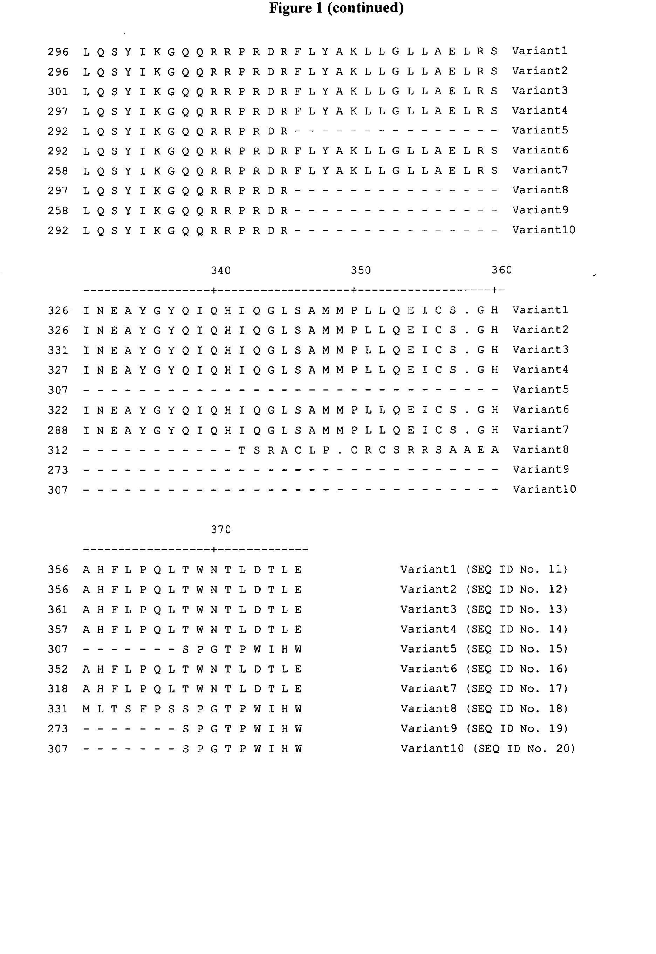

Figure 1 depicts the amino acid sequence alignment of the CARs according to the present invention based o the amino acid sequences;
SEQ ID NOs. 1 to 10 depict the nucleotide sequences encoding for the polypeptides according to the present invention, and
SEQ ID NOs. 11 to 20 depict the amino acid sequences of the polypeptides according to the present invention.

### EXAMPLES

### EXAMPLE 1: CLONING AND EXPRESSION OF THE CARS ACCORDING TO THE INVENTION

Construction of suitable vectors containing the desired coding and control sequences employs standard ligation and restriction techniques that are well understood in the art. Isolated plasmids, DNA sequences, were synthesized oligonucleotides were cleaved, tailored, and religated in the form desired.

Site-specific DNA cleavage was performed by treatment with the suitable restriction enzyme (or enzymes) under conditions that are generally understood in the art, and the particulars of which are specified by the manufacturer of these commercially available restriction enzymes.

See, e.g., New England Biolabs, Product Catalog. In general, about 1 µg of plasmid and/or DNA sequence was cleaved by one unit of enzyme in about 20 µl of buffer solution. Often excess of restriction enzyme was used to ensure complete digestion of the DNA substrate.
Incubation times of about one hour to two hours at about 37°C are workable, although variations are tolerable.

After each incubation, protein was removed by extraction with phenol/chloroform, and may be followed by ether extraction. The nucleic acid was recovered from aqueous fractions by precipitation with ethanol. If desired, size separation of the cleaved fragments was performed by polyacrylamide gel or agarose gel electrophoresis using standard techniques. A general description of size separations is found in Methods in Enzymology 65, 499-560 (1980).

Transformed host cells are cells which have been transformed or transfected with recombinant expression constructs made using recombinant DNA techniques and comprising mammalian nuclear receptor CARs encoding sequences. Preferred host cells for transient transfection are COS-7 cells. Transformed host cells may ordinarily express nuclear receptor CARs, but host cells transformed for purposes of cloning or amplifying nucleic acid hybridisation probe DNA need not express the nuclear CARs receptor. When expressed, the mammalian nuclear CARs receptor protein was typically located in the host cell membrane.

Cultures of cells derived from multicellular organisms are desirable hosts for recombinant nuclear receptor CARs protein synthesis. In principal, any higher eukaryotic cell culture is workable, whether from vertebrate or invertebrate culture. However, mammalian cells are preferred. Propagation of such cells in cell culture has become a routine procedure. See Tissue Culture (Academic Press, Kruse & Patterson, Eds., 1973). Examples of useful host cell lines are bacteria cells, insect cells, yeast cells, human 293 cells, VERO and HeLa cells, LMTK-cells, and WI138, BHK, COS-7, CV, and MDCK cell lines. Human 293 cells are preferred.

### EXAMPLE 2: NUCLEAR RECEPTOR CARS TISSUE LOCALIZATION:

A multiple tissue northern blot (Clontech, Palo Alto) was hybridised to a labelled probe. The blot contained about 03 to 3 µg of poly A RNA derived from various tissues. Hybridization was carried out in a hybridization solution such as one containing SSC (see Maniatis et al, ibid) at an optimized temperature between 50°c and 70°C, preferably 65°C. The filter was washed and a film exposed for signal detection (see also: Maniatis et al., *Molecular Cloning: A laboratory Manual,* Cold Spring Harbor Laboratory Press, N.Y.(1989)).

### EXAMPLE 3: NUCLEAR RECEPTOR CARS cDNA ISOLATION FROM HUMAN AND OTHER ORGANISMS:

A cloning strategy was used to clone the CARs receptor cDNA from specific cDNA libraries (Clontech, Palo Alto) or alternatively, RNA was obtained from various tissues and used to prepare cDNA expression libraries by using for example an Invitrogen kit. (Invitrogen Corporation, San Diego). For the isolation of the CARs cDNA clone the chosen library was screened under stringent condition (see definitions above) by using a CAR specific probe. The cDNA insert of positive clones was subsequently sequenced and cloned in a suitable expression vector.

Additionally, a full length receptor CARs clone from human can be obtained by using RACE PCR technology. In brief, suitable cDNA libraries were constructed or purchased. Following reverse transcription, the first strand cDNA was used directly in RACE PCR reactions using a RACE cDNA amplification kit according to the manufactures protocol (Clontech, Palo Alto). Amplified fragments were purified, cloned and subsequently used for sequence analysis.

### EXAMPLE 4: NUCLEAR RECEPTOR CARS LIGAND BINDING ASSAY:

The LBD of the receptor CAR was generated in a pure form as a fusion with glutathione-S-transferase (GST), and was used in an HTR-FRET assay together with the known cofactor SRC (amino acids 676-700). (see, for example, Warnmark A, et al.; Interaction of TIF2 NR-box peptides with the coactivator binding site of ERalpha. J Biol Chem 2002 Apr 5 Secondary reagents in the assay were Europium-labeled anti-GST antibody for the GST-CAR chimeric protein, and allphycocyanin-labeled streptavidin for the biotinylated peptide. Fluorescence was elicited at 320 nM and the ratio of the intensitiy of the emitted light at 665 and 615 nm was used as a measure of fluorescence resonance. The intensity of this readout at different concentrations of potential ligands was used as surrogate readout for receptor activation, reflected in enhanced cofactor peptided binding and thus enhanced fluorescence resonance. The buffer was 20 mM TRIS, pH 7.9, 2mM MgCl₂, 60 mM KCl, 0.8µg/µl bovine serum albumin, 1µM Europium-labeled anti-GST antibody, 8 ng/µl allophycocyanine-labeled streptavidin, 8 ng/µl recombinant GST-CAR-LBD protein, and a peptide from the human SRC protein (NH2 - CPSSHSSLTERHKILHRLLQEGSPS - COOH) at 0.8 ng/µl. Compounds were added as a solution in DMSO or ethanol (1µl). Assay volume was 25µl. Aftter a 60 minutes incubation in the dark at ambient temperature, the fluorescence was measured in a VictorV instrument (Wallac). The use of the different CARs allows the detection of the variant-specific activation using specific substances.

## Claims

1. A polypeptide of CAR, consisting essentially of one of the amino acid sequences according to SEQ ID Nos. 11 to 20, or having an amino acid sequence substantially the same or similar as SEQ ID Nos. 11 to 20.

2. A fusion protein comprising at least one polypeptide according to claim 1.

3. A polypeptide according to claim 1 or 2, wherein the polypeptide is a synthetically produced polypeptide.

4. A nucleic acid encoding for a polypeptide according to any of claims 1 to 3, wherein said nucleic acid is genomic DNA, cDNA, immature mRNA or mature mRNA, in particular derived from one of the nucleic acid sequences according to SEQ ID Nos. 1 to 10, optionally comprising a label.

5. A nucleic acid according to claim 4, wherein the sequence of the nucleic acid comprises at least one intron and/or a polyA-sequence.

6. A nucleic acid according to claim 4 or 5 in form of its complementary antisense-sequence.

7. A nucleic acid according to any of claims 4 to 6, wherein the nucleic acid is a synthetically produced nucleic acid.

8. A vector, in particular in form of a plasmid, shuttle vector, phagemid, cosmid, expression vector and/or vector for gene therapy, comprising a nucleic acid according to any of claims 4 to 7.

9. A host cell, comprising a vector according to claim 8.

10. A host cell according to claim 8 or 9, wherein the host cell expresses a polypeptide according to any of claims 1 to 3.

11. A method for the production of an antibody, in particular a monoclonal or polyclonal antibody, directed against a polypeptide according to any of claims 1 to 3, wherein an anti-dody producing organism is immunised with a polypeptide according to any of claims 1 to 3.

12. An antibody produced according to claim 11, **characterised in that** the antibody is directed against a polypeptide according to any of claims 1 to 3.

13. A method for screening for agents which are capable of modulating the cellular function of the CAR nuclear receptor, comprising the steps of:
contacting one or more candidate binding agents with a polypeptide according to any of claims 1 to 3,
removing unbound agent(s), and
detecting, whether the agent(s) interact with the polypeptide of the nuclear receptor.

14. A method according to claim 13, which comprises a FRET assay.

15. A method for modulating the cellular function of the CAR nuclear receptor, comprising the steps of:
contacting a cell with a binding agent of a polypeptide according to any of claims 1 to 3, whereby the cellular function of CAR nuclear receptor is modulated.

16. A method according to any of claims 13 to 15, wherein the modulation of the cellular function involves the ligand depending binding of a cofactor, e.g. TIF2 or other LBD-binding cofactors.

17. A method according to any of claims 13 to 16, **characterised in that** the binding agent is selected from the group comprising an antibody, RNA, an antisense oligonucleotide, steroids, a xenobiotic substance, and a ribozyme.

18. A method according to any of claims 15 to 17, **characterised in that** the cell is in a body.

19. Use of the CAR nuclear receptor sequence according to any of SEQ ID 1 to 20 for the prediction of an interaction of an agent which is capable of modulating the cellular function of the CAR nuclear receptor, in particular a medicament.
